(19) Europäisches Patentamt

European Patent Office

Office européen des brevets

(11) **EP 3 615 098 B1**

(12) **EUROPEAN PATENT SPECIFICATION**

(45) Date of publication and mention
of the grant of the patent:
**09.06.2021 Bulletin 2021/23**

(21) Application number: **18718482.5**

(22) Date of filing: **26.04.2018**

(51) Int Cl.:
*A61L 29/04* (2006.01)  *A61M 16/00* (2006.01)
*A61M 25/00* (2006.01)  *A61M 39/08* (2006.01)
*C08F 299/00* (2006.01)  *C08L 23/14* (2006.01)
*C08L 53/02* (2006.01)  *C08F 210/06* (2006.01)

(86) International application number:
**PCT/EP2018/060665**

(87) International publication number:
**WO 2018/197593 (01.11.2018 Gazette 2018/44)**

(54) **SOFT POLYPROPYLENE COMPOSITION WITH IMPROVED PROPERTIES**

WEICHE POLYPROPYLENZUSAMMENSETZUNG MIT VERBESSERTEN EIGENSCHAFTEN

COMPOSITION DE POLYPROPYLÈNE SOUPLE DOTÉE DE PROPRIÉTÉS AMÉLIORÉES

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **28.04.2017 EP 17168616**

(43) Date of publication of application:
**04.03.2020 Bulletin 2020/10**

(73) Proprietor: **Borealis AG**
**1220 Vienna (AT)**

(72) Inventors:
• **EK, Carl-Gustaf**
**426 58 Västra Frölunda (SE)**
• **GAHLEITNER, Markus**
**4021 Linz (AT)**
• **WANG, Jingbo**
**4021 Linz (AT)**
• **BERNREITNER, Klaus**
**4021 Linz (AT)**

(74) Representative: **Maiwald Patent- und
Rechtsanwaltsgesellschaft mbH
Elisenhof
Elisenstraße 3
80335 München (DE)**

(56) References cited:
**EP-A1- 2 386 602    WO-A1-2016/162359**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

The present invention relates to polypropylene composition with improved low temperature impact, low tensile modulus and low gel content as well as to its manufacture and use and to articles made out of the new composition.

Polymers, like polypropylene, are increasingly used in different demanding applications. At the same time there is a continuous search for tailored polymers which meet the requirements of these applications.

For example heterophasic propylene copolymers (HECOs or RAHECOs) are already widely used in industry for various applications. Such HECOs or RAHECOs comprise a matrix being either a propylene homopolymer (HECOs) or a random propylene copolymer (RAHECOs) in which an elastomeric copolymer is dispersed (rubber phase). Thus the polypropylene matrix contains (finely) dispersed inclusions being not part of the matrix and said inclusions contain the elastomeric copolymer. The term "inclusion" according indicates that the matrix and the inclusion form different phases within the heterophasic system, said inclusions are for instance visible by high resolution microscopy, like electron microscopy or atomic force microscopy, or can be identified by dynamic mechanical thermal analysis (DMTA). Specifically in DMTA the presence of a multiphase structure can be identified by the presence of at least two distinct glass transition temperatures.

Soft heterophasic propylene copolymers are also widely used in a lot of application fields, like sheets, soft touch products for automotive, medical tubes or pouches, foaming, roofing, bitumen modification, etc. However, due to the nature of the soft heterophasic propylene copolymers, the low temperature impact is poor because of the relatively high Tg of rubber phase (e.g. around -25°C to -50°C). This property consequently needs to be improved further. One way is to blend these materials with extra rubber materials, like styrene block copolymer or polyethylene plastomers, etc.. Here, however still the improvement is limited due to the factor that the compatibility issue between the external rubber and the matrix cannot be resolved easily.

A further way of to modify the soft heterophasic propylene copolymers is grafting. However, heterophasic propylene copolymers are particularly sensitive to degradation in particular in combination with peroxides, which limits the possibilities for modification by grafting.

In view of the above difficulties, there is still a desire to provide materials that do not have one or more of the mentioned disadvantages, in particular soft, flexible materials that have desirable mechanical and chemical properties, such as a low gel content, and easily tunable softness.

The one or other development to find suitable soft heterophasic propylene copolymers with the desired property profile was suggested in the past.

EP 2048185 describes soft PP compositions with soft touch feeling, said compositions comprising (A) a polypropylene, (B) an elastomeric copolymer of ethylene and a $C_3$-$C_8$ $\alpha$-olefin comonomer, and (C) an ethylene $C_2$-$C_8$ alkyl acrylate copolymer wherein the composition has a gel content of 0.25 to 1.00 wt% determined as the xylene hot insoluble fraction, whereby the polypropylene (A) can be the matrix of an heterophasic propylene copolymer (RAHECO) and the elastomeric copolymer (B) is an amorphous part of said heterophasic propylene copolymer (RAHECO).

It is preferred that said composition comprises additionally units (D) derived from at least bifunctionally unsaturated monomer(s) (D') and/or at least multifunctionally unsaturated low molecular weight polymer(s) (D"). The chemical nature of component (C) limits the practical applicability of the respective compositions, and the examples demonstrate the practical necessity of component (D), generating additional process complexity.

EP 1564231 A1 relates to the process of the grafting of monomers onto polyolefins in the presence of organic peroxides, whereby the monomer(s) and organic peroxide have been absorbed into a carrier polymer that, under the grafting conditions, undergoes chain scission in preference to cross-linking in the presence of organic peroxides. According to this patent application the described grafting process allows to obtain impact-modified, grafted polyolefin compounds with improved extrudability, heat stability and reduced gels or imperfections. The impact-modified, grafted polyolefins are obtained from ethylene polymer or copolymer (A), an impact modifier copolymer (B) and product (C) being a propylene polymer or copolymer into which has been incorporated organic peroxide and a polar monomer and antioxidants. As suitable impact modifiers (B) i.a. copolymer and/or a terpolymer containing styrene/butadiene/styrene (SBS) blocks, styrene/ethylene-butene/styrene (SEBS) blocks or styrene/isoprene/styrene (SIS) blocks or their hydrogenated or partially hydrogenated equivalents are mentioned, whereas in the examples polymer (B) is a pelletised ethylenepropylene copolymer.

The only property measured was the adhesion to aluminium foils.

Nevertheless no soft heterophasic propylene copolymers composition was disclosed so far which shows simultaneously improved low temperature impact, low tensile modulus and low gel content and is easy obtainable by an effective grafting process.

Accordingly it is the object of the present invention to provide such soft heterophasic propylene copolymers composition which fulfils all of the above mentioned requirements.

The finding of the present invention is that a heterophasic propylene copolymers must be grafted with a specific styrene based elastomer in order to achieve soft heterophasic propylene copolymers compositions which fulfil all of the above mentioned requirements.

## Summary of the Invention

Thus the present invention is related to grafted heterophasic propylene copolymer composition, which comprises

(A) 30.0 to 95.0 wt% of a heterophasic polypropylene copolymer having a melt flow rate (MFR 230°C/2.16kg, ISO1133) of 0.1 to 100 g/10min comprising

(a1) a matrix (M) being a crystalline polypropylene homo- or copolymer and
(a2) and elastomeric copolymer (E) dispersed in said matrix (M)

(B) 5.0 to 70.0 wt% of a styrene-based elastomer being a unsaturated styrene block copolymer containing at least one diene comonomer and having a styrene content of 5.0 to 50.0 wt% measured by Fourier transform infrared spectroscopy (FTIR),
and wherein the styrene-based elastomer (B) is grafted onto the heterophasic polypropylene copolymer (A), wherein the grafted heterophasic propylene copolymer composition has an impact strength measured according to the Charpy notched impact test according to ISO 179 (1eA) at -20°C of at least 15.0 kJ/m$^2$, impact strength measured according to the Charpy notched impact test according to ISO 179 (1eA) at -30°C of at least 7.0 kJ/m$^2$ and F30 melt strength of at least 3.0 cN measured with the Rheotens test at 200°C according to ISO 16790:2005.

Surprisingly it has been found out that such grafted heterophasic polypropylene copolymer compositions have superior properties compared to non-grafted compositions (see table 2). For instance such grafted heterophasic polypropylene copolymer composition has a low tensile modulus as well high impact strength, especially at low temperatures. In addition the grafted heterophasic polypropylene copolymer composition has a low amount of gels, being measured as the fraction insoluble in p-xylene at 25°C (XCI fraction).

In a further embodiment the present invention is related to a process for producing the grafted heterophasic polypropylene copolymer composition, which process is a reactive melt modification process, comprising the steps of

i) mixing component (A), the heterophasic polypropylene copolymer, with component (B), the styrene-based elastomer, and a thermally decomposing free radical-forming compound (C) and
ii) extruding said mixture.

In an additional embodiment the present invention is related to the use of the grafted heterophasic polypropylene copolymer composition for producing extruded films or tubes, blow moulded or injection moulded articles, and to the articles comprising the grafted heterophasic polypropylene copolymer composition.
Especially such articles are medical tubes or pouches comprising more than 90 wt% of the grafted heterophasic polypropylene copolymer composition.

## Detailed description

In the following the individual components are defined in more detail.
The grafted heterophasic propylene copolymer composition according to the present invention comprises

(A) 30.0 to 95.0 wt% of a heterophasic polypropylene copolymer having a melt flow rate (MFR 230°C/2.16kg, ISO1133) of 0.1 to 100 g/10min comprising
and
(B) 5.0 to 70.0 wt% of a styrene-based elastomer being a unsaturated styrene block copolymer containing at least one comonomer selected from butadiene and isoprene and having a styrene content of 5.0 to 50.0 wt%,
wherein the styrene-based elastomer (B) is grafted onto the heterophasic polypropylene copolymer (A), wherein the grafted heterophasic propylene copolymer composition has an impact strength measured according to the Charpy notched impact test according to ISO 179 (1eA) at -20°C of at least 15.0 kJ/m$^2$, impact strength measured according to the Charpy notched impact test according to ISO 179 (1eA) at -30°C of at least 7.0 kJ/m$^2$ and F30 melt strength of at least 3.0 cN measured with the Rheotens test at 200°C according to ISO 16790:2005.

The terms "heterophasic polypropylene copolymer", "base heterophasic polypropylene copolymer" used herein have the same meaning and are thus interchangeable. These terms stand for the non-grafted copolymer as such, i.e. without the styrene-based elastomer.

The term "grafted heterophasic propylene copolymer composition" used herein means the composition comprising the heterophasic propylene copolymer onto which the styrene-base elastomer is grafted.

*Component (A): base heterophasic polypropylene copolymer*

The base heterophasic polypropylene copolymer comprises at least

(a1) a matrix (M) being a crystalline polypropylene homo- or copolymer and
(a2) and elastomeric copolymer (E) dispersed in said matrix (M).

The term "heterophasic polypropylene copolymer" used herein denotes copolymers consisting of a matrix resin, being a crystalline polypropylene homo- or copolymer and an elastomeric, i.e. predominantly amorphous copolymer (E) dispersed in said matrix resin, as defined in more detail below.

Optionally it further comprises component (a3) being a crystalline copolymer fraction of ethylene with propylene or with a $CH_2=CHR$ alpha-olefin, and/or
(a4) a nucleating agent.

In the present invention, the term "matrix" is to be interpreted in its commonly accepted meaning, i.e. it refers to a continuous phase (in the present invention a continuous polymer phase) in which isolated or discrete particles such as rubber particles may be dispersed. The crystalline propylene polymer is present in such an amount that it forms a continuous phase which can act as a matrix.

Furthermore the terms "elastomeric copolymer", "dispersed phase" and "rubber phase" denote the same, i.e. are interchangeable.

*Ad component (a1):*

Component (a1) of the particular heterophasic polypropylene copolymer is a crystalline isotactic propylene homo- or copolymer forming the matrix of the heterophasic polypropylene copolymer.

The expression homopolymer used in the instant invention relates to a polypropylene that consists substantially, i.e. of at least 97.0 wt%, preferably of at least 98.0 wt%, more preferably of at least 99.0 wt%, still more preferably of at least 99.8 wt% of propylene units. In a preferred embodiment only propylene units in the propylene homopolymer are detectable.

The propylene homopolymer matrix is isotactic having a high pentad concentration, i.e. higher than 96.0 mol%, like a pentad concentration of at least 96.3 mol%. The pentad concentration is preferably 96.5 mol% up to 99.9% and more preferably 96.7 mol% to 99.8%.

The matrix can also be a propylene copolymer, i.e. a random propylene copolymer.

The random propylene copolymer comprises monomers copolymerizable with propylene, for example comonomers such as ethylene and/or $C_4$ to $C_8$ $\alpha$-olefins, in particular ethylene and/or $C_4$ to $C_6$ $\alpha$-olefins, e.g. 1-butene and/or 1-hexene. Preferably the random propylene copolymer comprises, especially consists of, monomers copolymerizable with propylene from the group consisting of ethylene, 1-butene and 1-hexene. More specifically the random propylene copolymer comprises - apart from propylene - units derivable from ethylene and/or 1-butene. In a preferred embodiment the random propylene copolymer comprises units derivable from ethylene and propylene only.

The random propylene copolymer is featured by a moderate comonomer content. Accordingly, the comonomer content of the random propylene copolymer is in the range of 1.0 to 12.0 wt%, preferably in the range of 2.0 to 10.0 wt% and more preferably in the range of 3.0 to 8.0 wt%.

The propylene homo- or copolymer matrix has a melt flow rate $MFR_2$ (ISO 1133; 230°C; 2.16kg) in the range of 0.5 to 100.0 g/10 min, preferably in the range of 1.0 to 50.0 g/10 min more preferably in the range of 1.5 to 20.0 g/10 min and even more preferably 2.0 to 10.0 g/10min.
The $MFR_2$ of the matrix is named matrix melt flow rate ($MFR_M$).

Moreover it is preferred that the amount of xylene solubles of propylene homo- or copolymer matrix is in a defined range. Xylene solubles are the part of the polymer soluble in cold xylene determined by dissolution in boiling xylene and letting the insoluble part crystallize from the cooling solution (determined at 25°C according to ISO 16152). The xylene solubles fraction contains polymer chains of low stereo-regularity and is an indication for the amount of non-crystalline areas. Accordingly it is preferred that the xylene solubles fraction of the propylene homo- or copolymer matrix is in the range of 0.5 wt% to 20.0 wt%, more preferably in the range of 0.7 wt% to 18.0 wt%. In an even more preferred embodiment the xylene solubles fraction is in the range of 0.8 wt% to 16.0 wt%.

The propylene homo- or copolymer matrix can be unimodal or multimodal, like bimodal.
When the propylene homo- or copolymer matrix phase is unimodal with respect to the molecular weight distribution, it may be prepared in a single stage process e.g. a slurry or gas phase process in a slurry or gas phase reactor. Preferably,

**EP 3 615 098 B1**

a unimodal matrix phase is polymerized as a slurry polymerization. Alternatively, the unimodal matrix may be produced in a multistage process using at each stage process conditions which result in similar polymer properties.

Concerning the definition of unimodal and multimodal, like bimodal, it is referred to the definition below.

Where the propylene homo- or copolymer matrix comprises two or more different propylene polymers these may be polymers with different monomer make up and/or with different molecular weight distributions and/or $MFR_2$. These components may have identical or differing monomer compositions and tacticities.

Thus in one embodiment of the present invention the matrix is unimodal, whereas in another embodiment the matrix (M) is bimodal and consists of two propylene homopolymer fractions, two propylene copolymers fractions or one homo and one copolymer fraction.

In one preferred embodiment the matrix of component (A) is a random propylene copolymer (R-PP). Even more preferably the matrix of component (A) is a random propylene-ethylene copolymer.

*Ad component (a2):*

Component (a2) of the particular heterophasic polypropylene copolymer is the elastomeric copolymer (E), which is a predominantly amorphous copolymer (ii) of ethylene and a $C_3$-$C_{10}$ $\alpha$-olefin.

Thus, component (a2) is an elastomeric copolymer, being dispersed in said matrix (M) (i.e. dispersed phase).

The elastomeric copolymer has an ethylene content, in polymerized form, in the range of from 20.0 to 80.0 wt%, preferably in the range from 20.0 to 60.0 wt% and more preferably in the range from 20.0 to 50.0 wt%.

The elastomeric copolymer (E) comprises, especially consists of, ethylene and comonomers copolymerizable with ethylene for example comonomers such as propylene and/or $C_4$ to $C_{10}$ $\alpha$-olefins, in particular propylene and/or $C_4$ to $C_6$ $\alpha$-olefins, e.g. 1-butene and/or 1-hexene, in polymerized form. Preferably, the elastomeric copolymer comprises, especially consists of, ethylene and comonomers copolymerizable with ethylene from the group consisting of propylene, 1-butene and 1-hexene. More specifically the elastomeric copolymer (E) comprises - apart from ethylene - units derivable from propylene and/or 1-butene. Thus in an especially preferred embodiment the elastomeric copolymer (E) comprises units derivable from ethylene and propylene only. It is especially preferred that the random propylene copolymer (R-PP) and the elastomeric copolymer (E) comprises the same comonomers in polymerized form.

Accordingly in one specific embodiment the matrix random propylene copolymer (R-PP) and the elastomeric copolymer (E) comprise propylene and ethylene, in polymerized form, only.

As stated above, the terms "elastomeric copolymer", "dispersed phase" and "rubber phase" denote the same, i.e. are interchangeable.

Like the propylene homo- or copolymer matrix, the dispersed phase can be unimodal or multimodal, like bimodal.

In one embodiment, the dispersed phase is unimodal. More particularly, the dispersed phase is preferably unimodal in view of the intrinsic viscosity and/or the comonomer distribution. Concerning the definition of unimodal and multimodal, like bimodal, it is referred to the definition above.

Preferably the unimodal dispersed phase is made in one reaction stage, more preferably in a gas phase reactor ad comprises, respectively consists of one copolymer fractions.

*Ad component* (a3)

As component (a3) a crystalline ethylene copolymer fraction of ethylene with an $\alpha$-olefin with 3-10 carbon atoms is optionally present, said fraction being insoluble in xylene at ambient temperature.

$\alpha$-olefins with 3-10 carbon atoms are for example propylene, 1-butene, 1-pentene, 1-hexene, 1-heptene and 1-octene.

The crystalline ethylene copolymer is a by-reaction product obtained by the preparation of the heterophasic polypropylene copolymer. Such crystalline ethylene copolymer is present as inclusion in the amorphous phase due to thermodynamic reasons.

The crystalline ethylene copolymer has a melting temperature Tm2 and a melting enthalpy Hm2 as determined by DSC analysis according to ISO 11357.

Preferably, Tm2 of the crystalline ethylene copolymer is within the range of 105°C to 130°C, more preferably within the range of 110°C to 127°C and most preferably within the range of 112°C to 124°C.

Preferably, Hm2 of the crystalline ethylene copolymer is less than 4.0 J/g, more preferably less than 2.0 J/g and most preferably less than 1.0 J/g.

The crystalline ethylene copolymer is present in an amount from 0.0 to 5.0 wt%

Ad component (a4)

As optional component (a-4) a nucleating agent (NA) for promoting the $\alpha$-phase of isotactic polypropylene can be present, i.e. an $\alpha$-nucleating agent.

The $\alpha$-nucleating agent is preferably selected from the groups consisting of

(i) salts of monocarboxylic acids and polycarboxylic acids, e.g. sodium benzoate or aluminum tert-butylbenzoate, and

(ii) dibenzylidenesorbitol (e.g. 1,3 : 2,4 dibenzylidenesorbitol) and C1-C8-alkyl-substituted dibenzylidenesorbitol derivatives, such as methyldibenzylidenesorbitol, ethyldibenzylidenesorbitol or dimethyldibenzylidenesorbitol (e.g. 1,3 2,4 di(methylbenzylidene) sorbitol), or substituted nonitol-derivatives, such as 1,2,3,-trideoxy-4,6:5,7-bis-O-[(4-propylphenyl)methylene]-nonitol, and

(iii) salts of diesters of phosphoric acid, e.g. sodium 2,2'-methylenebis (4, 6,-di-tert-butylphenyl) phosphate or aluminium-hydroxy-bis[2,2'-methylene-bis(4,6-di-t-butylphenyl)phosphate], and

(iv) polymeric nucleating agent, and

(v) mixtures thereof.

Such additives are generally commercially available and are described, for example, in "Plastic Additives Handbook", 5th edition, 2001 of Hans Zweifel.

Preferably the $\alpha$-nucleating agent is a polymeric nucleating agent, more preferably a vinylcycloalkane polymer and/or a vinylalkane polymer.

Said polymeric nucleating agent may be introduced into the heterophasic copolymer by blending with a masterbatch (MB) together with e.g. a carrier polymer or during polymerization of the heterophasic propylene copolymer, preferably, the polymeric nucleating agent is introduced into the composition by prepolymerizing the catalyst used to prepare a part or all of the heterophasic propylene copolymer.

Any known polymeric nucleating agent may be employed including polymers of vinyl alkanes and vinyl cycloalkanes.

A preferred example of such a polymeric nucleating agent is a vinyl polymer, such as a vinyl polymer derived from monomers of the formula

$$CH_2 = CH-CHR^1R^2$$

wherein $R^1$ and $R^2$, together with the carbon atom they are attached to, form an optionally substituted saturated or unsaturated or aromatic ring or a fused ring system, wherein the ring or fused ring moiety contains four to 20 carbon atoms, preferably 5 to 12 membered saturated or unsaturated or aromatic ring or a fused ring system or independently represent a linear or branched $C_4$-$C_{30}$ alkane, $C_4$-$C_{20}$ cycloalkane or $C_4$-$C_{20}$ aromatic ring. Preferably $R^1$ and $R^2$, together with the C-atom wherein they are attached to, form a five- or six-membered saturated or unsaturated or aromatic ring or independently represent a lower alkyl group comprising from 1 to 4 carbon atoms. Preferred vinyl compounds for the preparation of a polymeric nucleating agent to be used in accordance with the present invention are in particular vinyl cycloalkanes, in particular vinyl cyclohexane (VCH), vinyl cyclopentane, and vinyl-2-methyl cyclohexane, 3-methyl-1-butene, 3-ethyl-1-hexene, 3-methyl-1-pentene, 4-methyl-1-pentene or mixtures thereof. VCH is a particularly preferred monomer.

In case the nucleating agent is incorporated to the heterophasic propylene copolymer in the form of a masterbatch (MB) said polymeric nucleating agent, which is preferably alpha-nucleating agent, most preferably a vinylcycloalkane, like vinylcyclohexane (VCH), polymer and/or vinylalkane polymer, preferably vinylcyclohexane (VCH), as defined above or below, is preferably present in an amount of not more than 500 ppm, more preferably of 1 to 200 ppm, and still more preferably of 5 to 100 ppm, based on the weight of the master batch (100 wt%). In this embodiment, more preferably, said masterbatch (MB) is present in an amount of not more than 10.0 wt%, more preferably not more than 5.0 wt% and most preferably not more than 3.5 wt%, with the preferred amount of masterbatch (MB) being from 1.5 to 3.5 wt%, based on the total amount of the heterophasic propylene copolymer. Most preferably the masterbatch (MB) comprises, preferably consists of a homopolymer or copolymer, preferably homopolymer, of propylene which has been nucleated according to BNT-technology as described below.

It is preferred that the polymeric nucleating agent is introduced to the heterophasic propylene copolymer during the polymerization process of the heterophasic propylene copolymer. The nucleating agent is preferably introduced to the heterophasic propylene copolymer by first polymerizing the above defined vinyl compound, preferably vinylcycloalkane, as defined above or below, in the presence of a catalyst system comprising a solid catalyst component, preferably a solid Ziegler Natta catalyst component, a cocatalyst and optional external donor, and the obtained reaction mixture of the polymer of the vinyl compound, preferably vinyl cyclohexane (VCH) polymer, and the catalyst system is then used for producing the heterophasic propylene copolymer. The above incorporation of the polymeric nucleating agent to the heterophasic propylene copolymer during the polymerization of said heterophasic propylene copolymer is called herein as BNT-technology as described below.

Said obtained reaction mixture is herein below referred interchangeably as modified catalyst system.

Preferably the polymeric nucleating agent is vinylcyclohexane (VCH) polymer which is introduced into the heterophasic propylene copolymer by the BNT technology.

More preferably in this preferred embodiment, the amount of polymeric nucleating agent, like vinylcyclohexane (VCH), polymer and/or vinylalkane polymer, more preferably of vinylcyclohexane (VCH) polymer, in the heterophasic propylene copolymer is not more than 500 ppm, more preferably of 1 to 200 ppm, most preferably 5 to 100 ppm.

With regard to the BNT-technology reference is made to the international applications WO 99/24478, WO 99/24479 and particularly WO 00/68315. According to this technology a catalyst system, preferably a Ziegler-Natta procatalyst, can be modified by polymerizing a vinyl compound in the presence of the catalyst system, comprising in particular the Ziegler-Natta procatalyst, an external donor and a cocatalyst, which vinyl compound has the formula: $CH_2=CH-CHR^1R^2$ as defined above.

The polymerized vinyl compound acts as an alpha-nucleating agent. The weight ratio of vinyl compound to solid catalyst component in the modification step of the catalyst is preferably of up to 5 (5:1), preferably up to 3 (3:1) most preferably from 0.5 (1:2) to 2 (2:1). The most preferred vinyl compound is vinylcyclohexane (VCH).

When a polymeric nucleating agent is introduced to the heterophasic propylene copolymer during the polymerization process, the amount of nucleating agent present in the heterophasic propylene copolymer is preferably not more than 500 ppm, more preferably is 0.025 to 200 ppm, still more preferably is 1 to 100 ppm, and most preferably is 5 to 100 ppm, based on the heterophasic propylene copolymer and the nucleating agent, preferably based on the total weight of the heterophasic propylene copolymer including all additives.

*Properties of Component (A), the base heterophasic polypropylene copolymer*

The heterophasic polypropylene copolymer of the present inventions is further characterized by a total melt flow rate ($MFR_T$) (ISO 1133; 230°C; 2.16kg) in the range of 0.1 to 100.0 g/10 min, preferably in the range of 1.0 to 80.0 g/10 min, more preferably in the range of 2.0 to 60.0 g/10 min and even more preferably in the range of 4.0 to 40.0 g/10 min.

Preferably the final melt flow rate of the heterophasic propylene copolymer is adjusted during the polymerization process. Accordingly the reactor-made heterophasic propylene copolymer has the melt flow rate as defined above or in the claims. "Reactor-made heterophasic propylene copolymer" denotes herein that the melt flow rate of the heterophasic propylene copolymer has not been modified on purpose by post-treatment. Accordingly, in a preferred embodiment the heterophasic propylene copolymer is non-visbroken, particularly not visbroken using peroxide. Accordingly, the melt flow rate is not increased by shortening the chain length of the heterophasic propylene copolymer according to this invention by use of peroxide. Thus it is preferred that the heterophasic propylene copolymer does not contain any peroxide and/or decomposition product thereof.

It is also appreciated that the total content of the ethylene comonomers in the total heterophasic propylene copolymer is rather moderate.

Accordingly it is preferred that the heterophasic propylene copolymer has a total ethylene comonomer content in the range of 2.5 to 15.0 wt%,wt%, preferably in the range of 3.0 to 12.0 wt% and more preferably in the range of 4.0 to 10.0 wt%.

Furthermore the inventive heterophasic polypropylene copolymer has at least a first glass transition temperature Tg(1) and a second glass transition temperature Tg(2), wherein said first glass transition temperature Tg(1) is above the second glass transition temperature Tg(2). The glass transition temperature Tg is determined by dynamic mechanical analysis (DMTA) according to ISO 6721-7.

Accordingly it is especially preferred that the heterophasic polypropylene copolymer has a first glass transition temperature Tg(1) in the range of -12°C to +4°C and/or a second glass transition temperature Tg(2) in the range of -65°C to -30°C.

The multiphase structure of the heterophasic polypropylene copolymer (predominantly amorphous ethylene copolymer dispersed in the matrix) can be identified by the presence of at least two distinct glass transition temperatures. The higher first glass transition temperature (Tg(1)) represents the matrix, i.e. the crystalline polypropylene homo- or copolymer, whereas the lower second glass transition temperature (Tg(2)) reflects the predominantly amorphous ethylene copolymer of the heterophasic polypropylene copolymer.

Preferably the first glass transition temperature Tg(1) is in the range of -8°C to +3°C, more preferably in the range of -5°C to +2.0°C.

The second glass transition temperature Tg(2) is preferably in the range of -62°C to -40°C, more preferably in the range of -60°C to -45°C.

The amount of the xylene cold soluble (XCS) fraction of the heterophasic propylene copolymer is in the range of 10.0 to 50.0 wt% in view of the base heterophasic propylene copolymer, preferably in the range of 10.0 to 40.0 wt% and more preferably in the range of 10.0 to 30.0 wt%.

The intrinsic viscosity (IV) measured according to ISO 1628-1 (at 135°C in decaline) of the XCS fraction of the heterophasic propylene copolymer is in the range of more than 1.0 to 5.0 dl/g, preferably in the range of 1.2 to 4.0 dl/g and more preferably in the range of 1.3 to 3.0 dl/g.

The heterophasic propylene copolymer of the present invention is composed of components (a1) and (a2) and optional components (a3) and/or (a4).

Component (a1) is present in an amount of from 50.0 to 90.0 wt%, preferably from 52.0 to 88.0 wt% and more preferably from 54.0 to 85.0 wt%.

Component (a2) is present in an amount of from 10.0 to 50.0 wt%, preferably from 12.0 to 48.0 wt% and more preferably from 15.0 to 46.0 wt%.

Component (a3) is present in an amount of from 0 to 5.0 wt%, preferably from 0.0 to 4.0 wt% and more preferably from 0.0 to 3.0 wt%.

Component (a4) is present in an amount of from 0.0 to 1.0 wt%, preferably from 0.0 to 0.5 wt% and more preferably from 0.0 to 0.1 wt%.

If component (a4) is added in the form of a masterbatch (MB), the amount of masterbatch containing component (a4) is up to 10 wt% related to the entire heterophasic propylene copolymer, preferably up to 5 wt% and more preferably in the range of 1.5 to 3.5 wt% based on the entire heterophasic propylene copolymer.

The sum of fractions (a1), (a2), (a3) and (a4) is 100 wt% or lower depending on the presence of further fractions or additives. The ranges in percent by weight (wt%) as used herein define the amount of each of the fractions or components based on the entire heterophasic propylene copolymer according to the present invention. All fractions and components together give a sum of 100 wt%.

The heterophasic propylene copolymer according to the present invention apart from the polymeric components and the nucleating agent (a4), optionally in the form of a masterbatch (MB), may comprise further non-polymeric components, e.g. additives for different purposes.

The following are optional additives: process and heat stabilisers, pigments and other colouring agents allowing retaining transparency, antioxidants, antistatic agents, slip agents, antiblocking agent, UV stabilisers and acid scavengers.

Depending on the type of additive, these may be added in an amount of 0.001 to 2.0 wt%, based on the weight of the heterophasic propylene copolymer.

*Preparation of the heterophasic propylene copolymer*

The heterophasic propylene copolymer in accordance with the present invention may be prepared by any suitable process, including in particular blending processes such as mechanical blending including mixing and melt blending processes and any combinations thereof as well as in-situ blending during the polymerization process. These can be carried out by methods known to the skilled person, including batch processes and continuous processes. The heterophasic propylene copolymer according to the invention is preferably prepared by a sequential polymerization process, as described below, in the presence of a catalyst system comprising a Ziegler-Natta Catalyst (ZN-C), a cocatalyst (Co) and optionally an external donor (ED), as described below.

The term "sequential polymerization system" according to this invention indicates that the heterophasic propylene copolymer is produced in at least two polymerization reactors connected in series. Accordingly, the present polymerization system comprises at least a first polymerization reactor (R1), a second polymerization reactor (R2), optionally a third polymerization reactor (R3), and further optionally a fourth polymerization reactor (R4). The term "polymerization reactor" shall indicate that the main polymerization takes place. Preferably, at least one of the two polymerization reactors (R1) and (R2) is a gas phase reactor (GPR). More preferably the second polymerization reactor (R2), the optional third polymerization reactor (R3) and the optional fourth polymerization reactor (R4) are gas phase reactors (GPRs), i.e. a first gas phase reactor (GPR1) and a second gas phase reactor (GPR2) and a third gas phase reactor (GPR3). A gas phase reactor (GPR) according to this invention is preferably a fluidized bed reactor, a fast fluidized bed reactor or a settled bed reactor or any combination thereof.

Accordingly, the first polymerization reactor (R1) is preferably a slurry reactor (SR) and can be any continuous or simple stirred batch tank reactor or loop reactor operating in bulk or slurry. Bulk means a polymerization in a reaction medium that comprises of at least 60 % (w/w) monomer. According to the present invention the slurry reactor (SR) is preferably a (bulk) loop reactor (LR).

In this first polymerization reactor (R1) the matrix propylene homo-or copolymer or part of it, i.e. a first propylene homo-or copolymer fraction, is produced.

Preferably this propylene homo- or copolymer of the first polymerization reactor (R1), more preferably the polymer slurry of the loop reactor (LR) containing the matrix (M) or part of it, i.e. a first propylene homo-or copolymer fraction, is directly fed into the second polymerization reactor (R2), i.e. into the (first) gas phase reactor (GPR1), without a flash step between the stages.

This kind of direct feed is described in EP 887379 A, EP 887380 A, EP 887381 A and EP 991684 A.

By "direct feed" is meant a process wherein the content of the first polymerization reactor (R1), i.e. of the loop reactor (LR), the polymer slurry comprising the propylene homo- or copolymer matrix or part of it, i.e. a first propylene homo- or copolymer fraction, is led directly to the next stage gas phase reactor.

Alternatively, the propylene homo- or copolymer of the first polymerization reactor (R1), preferably polymer slurry of the loop reactor (LR) containing the propylene homo- or copolymer matrix or part of it, may be also directed into a flash step or through a further concentration step before fed into the second polymerization reactor (R2), i.e. into the 1st gas phase reactor (GPR1). Accordingly, this "indirect feed" refers to a process wherein the content of the first polymerization reactor (R1), of the loop reactor (LR), i.e. the polymer slurry, is fed into the second polymerization reactor (R2), into the (first) gas phase reactor (GPR1), via a reaction medium separation unit and the reaction medium as a gas from the separation unit.

More specifically, the second polymerization reactor (R2) and any subsequent reactor, for instance, the third (R3) or fourth polymerization reactor (R4) are preferably gas phase reactors (GPRs). Such gas phase reactors (GPR) can be any mechanically mixed or fluid bed reactors. Preferably the gas phase reactors (GPRs) comprise a mechanically agitated fluid bed reactor with gas velocities of at least 0.2 m/sec. Thus it is appreciated that the gas phase reactor is a fluidized bed type reactor preferably with a mechanical stirrer.

Thus, in a preferred embodiment the first polymerization reactor (R1) is a slurry reactor (SR), like loop reactor (LR), whereas the second polymerization reactor (R2) and the optional third polymerization reactor (R3), and the optional fourth polymerization reactor (R4) are gas phase reactors (GPRs).

Accordingly for the instant process at least two, preferably two polymerization reactors (R1), and (R2) or three polymerization reactors (R1), (R2) and (R3), or even four polymerization reactors (R1), (R2), R(3) and (R4), namely a slurry reactor (SR), like loop reactor (LR) and a (first) gas phase reactor (GPR1), an optional second gas phase reactor (GPR2), and optionally a third gas phase reactor (GPR3) connected in series are used.

Prior to the slurry reactor (SR) a pre-polymerization reactor is placed.
As the process covers also a pre-polymerization step, all of the Ziegler-Natta catalyst (ZN-C) is fed in the pre-polymerization reactor. Subsequently the pre-polymerization product containing the Ziegler-Natta catalyst (ZN-C) is transferred into the first polymerization reactor (R1).

Especially good results are achieved in case the temperature in the reactors is carefully chosen.
Accordingly it is preferred that the operating temperature in the first polymerization reactor (R1) is in the range of 62 to 85°C, more preferably in the range of 65 to 82°C, still more preferably in the range of 67 to 80°C.
Alternatively or additionally to the previous paragraph it is preferred that the operating temperature in the second polymerization reactor (R2) and in the optional third reactor (R3) and the optional the fourth reactor (R4) is in the range of 75 to 95°C, more preferably in the range of 78 to 92°C.
Preferably the operating temperature in the second polymerization reactor (R2) is equal to or higher than the operating temperature in the first polymerization reactor (R1). Accordingly it is preferred that the operating temperature

(a) in the first polymerization reactor (R1) is in the range of 62 to 85°C, more preferably in the range of 65 to 82°C, still more preferably in the range of 67 to 80°C, like 70 to 80°C;
and
(b) in the second polymerization reactor (R2) is in the range of 75 to 95°C, more preferably in the range of 78 to 92°C, still more preferably in the range of 78 to 88°C,
with the proviso that the operating temperature in the in the second polymerization reactor (R2) is equal or higher to the operating temperature in the first polymerization reactor (R1).

Typically the pressure in the first polymerization reactor (R1), preferably in the loop reactor (LR), is in the range from 20 to 80 bar, preferably 30 to 70 bar, like 35 to 65 bar, whereas the pressure in the second polymerization reactor (R2), i.e. in the (first) gas phase reactor (GPR1), and in any subsequent reactor, like in the third polymerization reactor (R3), e.g. in the second gas phase reactor (GPR2), or in a fourth polymerization reactor (R4), e.g. in the third gas phase reactor (GPR3) is in the range from 5 to 50 bar, preferably 15 to 40 bar.
Preferably hydrogen is added in each polymerization reactor in order to control the molecular weight, i.e. the melt flow rate $MFR_2$.

Preferably the average residence time is rather long in the polymerization reactors (R1) and (R2). In general, the average residence time ($\tau$) is defined as the ratio of the reaction volume (VR) to the volumetric outflow rate from the reactor (Qo) (i.e. VR/Qo), i.e $\tau$ = VR/Qo [tau = VR/Qo]. In case of a loop reactor the reaction volume (VR) equals to the reactor volume.

Accordingly the average residence time ($\tau$) in the first polymerization reactor (R1) is preferably at least 5 min, more preferably in the range of 15 to 80 min, still more preferably in the range of 20 to 60 min, like in the range of 24 to 50 min, and/or the average residence time ($\tau$) in the second polymerization reactor (R2) is preferably at least 70 min, more preferably in the range of 70 to 220 min, still more preferably in the range of 80 to 210 min, yet more preferably in the range of 90 to 200 min, like in the range of 90 to 190 min. Preferably the average residence time ($\tau$) in the third polymerization reactor (R3) or in the fourth polymerization reactor (R4) - if present- is preferably at least 30 min, more preferably in the range of 30 to 120 min, still more preferably in the range of 40 to 100 min, like in the range of 50 to 90 min.

As mentioned above the preparation of the heterophasic propylene copolymer comprises preferably in addition to the (main) polymerization of the propylene polymer in the at least two polymerization reactors (R1, R2 and optional R3, R4) prior thereto a pre-polymerization in a pre-polymerization reactor (PR) upstream to the first polymerization reactor (R1).

In the pre-polymerization reactor (PR) a polypropylene (Pre-PP) is produced. The prepolymerization is conducted in the presence of the Ziegler-Natta catalyst (ZN-C). According to this embodiment the Ziegler-Natta catalyst (ZN-C), the co-catalyst (Co), and the external donor (ED) are all introduced to the pre-polymerization step. However, this shall not exclude the option that at a later stage for instance further co-catalyst (Co) and/or external donor (ED) is added in the polymerization process, for instance in the first reactor (R1). In one embodiment the Ziegler-Natta catalyst (ZN-C), the co-catalyst (Co), and the external donor (ED) are only added in the pre-polymerization reactor (PR).

The pre-polymerization reaction is typically conducted at a temperature of 10 to 60°C, preferably from 15 to 50°C, and more preferably from 20 to 45°C.

The pressure in the pre-polymerization reactor is not critical but must be sufficiently high to maintain the reaction mixture in liquid phase. Thus, the pressure may be from 20 to 100 bar, for example 30 to 70 bar.

In a preferred embodiment, the pre-polymerization is conducted as bulk slurry polymerization in liquid propylene, i.e. the liquid phase mainly comprises propylene, with optionally inert components dissolved therein. Furthermore, according to the present invention, an ethylene feed can be employed during pre-polymerization as mentioned above.

It is possible to add other components also to the pre-polymerization stage. Thus, hydrogen may be added into the pre-polymerization stage to control the molecular weight of the polypropylene (Pre-PP) as is known in the art. Further, antistatic additive may be used to prevent the particles from adhering to each other or to the walls of the reactor.

The precise control of the pre-polymerization conditions and reaction parameters is within the skill of the art.

Due to the above defined process conditions in the pre-polymerization, a mixture of the Ziegler-Natta catalyst (ZN-C) and the polypropylene (Pre-PP) produced in the pre-polymerization reactor (PR) is obtained. Preferably the Ziegler-Natta catalyst (ZN-C) is (finely) dispersed in the polypropylene (Pre-PP). In other words, the Ziegler-Natta catalyst (ZN-C) particles introduced in the pre-polymerization reactor (PR) split into smaller fragments which are evenly distributed within the growing polypropylene (Pre-PP). The sizes of the introduced Ziegler-Natta catalyst (ZN-C) particles as well as of the obtained fragments are not of essential relevance for the instant invention and within the skilled knowledge.

Accordingly, the heterophasic propylene copolymer is preferably produced in a process comprising polymerizing propylene in at least two subsequent polymerization steps in the presence of a Ziegler-Natta catalyst, whereby:

a) in the first polymerization reactor (R1), i.e. in a loop reactor (LR), propylene is polymerized optionally in the presence of a comonomer, obtaining either a first propylene homo- or copolymer fraction or the propylene homo- or copolymer matrix (M), transferring said first propylene homo- or copolymer fraction or the propylene homo- or copolymer matrix (M) to a second polymerization reactor (R2),

b) in the second polymerization reactor (R2)
either a second propylene homo- or copolymer fraction, forming together with the first propylene homo- or copolymer fraction the propylene homo- or copolymer matrix (M), or the predominantly amorphous ethylene copolymer fraction (a2) in the presence of the propylene homo- or copolymer matrix (M) produced in earlier step a) is produced, whereupon

c) if in the first and the second polymerization reactor the propylene homo- or copolymer matrix (M) is produced, in a third polymerization reactor (R3) the predominantly amorphous ethylene copolymer fraction (a2) in the presence of the propylene homo- or copolymer matrix (M) produced in earlier steps a) and b) is produced.

In another preferred process also a prepolymerization step is included prior to the reaction in the first polymerization reactor (R1).

In such a pre-polymerization step prior to the reaction in the first polymerization reactor (R1), a mixture of the Ziegler-Natta catalyst (ZN-C) and the polypropylene (Pre-PP) produced in the pre-polymerization reactor (PR) is obtained. Preferably the Ziegler-Natta catalyst (ZN-C) is (finely) dispersed in the polypropylene (Pre-PP). Subsequent to the pre-polymerization, the mixture of the Ziegler-Natta catalyst (ZN-C) and the polypropylene (Pre-PP) produced in the pre-polymerization reactor (PR) is transferred to the first reactor (R1). Typically the total amount of the polypropylene (Pre-PP) in the final heterophasic propylene copolymer (RAHECO) is rather low and typically not more than 5.0 wt%, more preferably not more than 4.0 wt%, still more preferably in the range of 0.5 to 4.0 wt%, like in the range 1.0 of to 3.0 wt%

A preferred multistage process is a "loop-gas phase"-process, as developed by Borealis (known as BORSTAR® technology) and is described e.g. in patent literature, such as in EP 0 887 379, WO 92/12182 WO 2004/000899, WO 2004/111095, WO 99/24478, WO 99/24479 or in WO 00/68315.

A further suitable slurry-gas phase process is the Spheripol® process of Basell.

The catalyst components are preferably all introduced to the prepolymerization step. However, where the solid catalyst component (i) and the cocatalyst (ii) can be fed separately it is possible that only a part of the cocatalyst is introduced into the prepolymerization stage and the remaining part into subsequent polymerization stages. Also in such cases it is necessary to introduce so much cocatalyst into the prepolymerization stage that a sufficient polymerization reaction is obtained therein.

It is possible to add other components also to the prepolymerization stage. Thus, hydrogen may be added into the prepolymerization stage to control the molecular weight of the prepolymer as is known in the art. Further, antistatic additive may be used to prevent the particles from adhering to each other or to the walls of the reactor.

The precise control of the prepolymerization conditions and reaction parameters is within the skill of the art.

According to the invention the heterophasic polypropylene composition is obtained by a multistage polymerization process, as described above, in the presence of a catalyst system.

In the process described above a Ziegler-Natta catalyst (ZN-C) for the preparation of the heterophasic polypropylene composition is applied. This Ziegler-Natta catalyst (ZN-C) can be any stereo-specific Ziegler-Natta catalyst (ZN-C) for propylene polymerization, which preferably is capable of catalysing the polymerization and copolymerization of propylene and comonomers at a pressure of 500 to 10000 kPa, in particular 2500 to 8000 kPa, and at a temperature of 40 to 110°C, in particular of 60 to 110°C.

Preferably, the Ziegler-Natta catalyst (ZN-C) comprises a high-yield Ziegler-Natta type catalyst including an internal donor component, which can be used at high polymerization temperatures of 80°C or more.

Such high-yield Ziegler-Natta catalyst (ZN-C) can comprise a succinate, a diether, a phthalate etc., or mixtures therefrom as internal donor (ID) and are for Example commercially available for example from LyondellBasell under the Avant ZN trade name.

Especially useful solid catalysts are also those disclosed in WO-A-2003/000757, WO-A-2003/000754, WO-A-2004/029112 and WO2007/137853. These catalysts are solid catalysts of spherical particles with compact structure and low surface area of the particles. Further, these catalysts are featured by a uniform distribution of catalytically active sites thorough the catalyst particles. Catalysts are prepared by emulsion-solidification method, where no external support is needed. The dispersed phase in the form of liquid droplets of the emulsion forms the catalyst part, which is transformed to solid catalyst particles during the solidification step. Thus, in an especially preferred embodiment of the present invention, the solid catalyst component is prepared by a process comprising:

- preparing a solution of magnesium complex by reacting an alkoxy magnesium compound and an electron donor or a precursor thereof in a C6-C10 aromatic liquid reaction medium;
- reacting said magnesium complex with a four valent titanium compound, preferably TiCl4 , at a temperature greater than 10 °C and less than 50 °C to produce an emulsion of a denser, dispersed phase having Ti/Mg mol ratio 0.1 to 10 and in a continuous phase having Ti/Mg mol ratio 10 to 100; and
- agitating the emulsion, optionally in the presence of an emulsion stabilizer and/or a turbulence minimizing agent, in order to maintain the droplets of said dispersed phase within an average size range of 5 to 200 μm.

The catalyst particles are obtained after solidifying said droplets of the dispersed phase by heating, preferably at a temperature from 80 °C to 110 °C. In said process an aluminium alkyl compound of the formula AIR3-nXn, where R is an alkyl and/or an alkoxy group of 1 to 20, preferably of 1 to 10 carbon atoms, X is a halogen and n is 0, 1 or 2 , is added and brought into contact with the droplets of the dispersed phase of the agitated emulsion. Alternatively, the aluminium alkyl compound of the formula AIR3-nXn, is brought into contact with the solidified particles at the washing step before recovering the final solid particles.

Suitable internal electron donors are, among others, (di)esters of aromatic (di)carboxylic acids. Said aromatic carboxylic acid ester or diester can be formed in situ by reaction of an aromatic carboxylic acid chloride or diacid chloride with a C2 - C16 alkanol and/or diol, and is preferable di-2-ethyl-hexyl phthalate.

A further suitable catalyst for the present invention is a solid Ziegler-Natta catalyst, which comprises compounds of a transition metal of Group 4 to 6 of IUPAC, like titanium, a Group 2 metal compound, like a magnesium, and an internal donor being a non-phthalic compound, more preferably a non-phthalic acid ester, still more preferably being a diester of non-phthalic dicarboxylic acids as described in more detail below. Thus, the catalyst is fully free of undesired phthalic compounds. Further, the solid catalyst is free of any external support material, like silica or MgCl2, but the catalyst is self-supported.

The Ziegler-Natta catalyst (ZN-C) can be further defined by the way as obtained.

Accordingly, the Ziegler-Natta catalyst (ZN-C) is preferably obtained by a process comprising the steps of

a)

a$_1$) providing a solution of at least a Group 2 metal alkoxy compound (Ax) being the reaction product of a Group 2 metal compound and a monohydric alcohol (A) comprising in addition to the hydroxyl moiety at least one ether moiety optionally in an organic liquid reaction medium; or

a$_2$) a solution of at least a Group 2 metal alkoxy compound (Ax') being the reaction product of a Group 2 metal compound and an alcohol mixture of the monohydric alcohol (A) and a monohydric alcohol (B) of formula ROH, optionally in an organic liquid reaction medium; or

a$_3$) providing a solution of a mixture of the Group 2 alkoxy compound (Ax) and a Group 2 metal alkoxy compound (Bx) being the reaction product of a Group 2 metal compound and the monohydric alcohol (B), optionally in an organic liquid reaction medium; or

$a_4$) providing a solution of Group 2 alkoxide of formula $M(OR_1)_n(OR_2)_m X_{2-n-m}$ or mixture of Group 2 alkoxides $M(OR_1)_{n'} X_{2-n'}$ and $M(OR_2)_{m'} X_{2-m'}$, where M is Group 2 metal, X is halogen, $R^1$ and $R_2$ are different alkyl groups of $C_2$ to $C_{16}$ carbon atoms, and $0 \leq n < 2$, $0 \leq m < 2$ and $n+m+(2-n-m) = 2$, provided that both n and m $\neq$ 0, 0 < $n' \leq 2$ and $0 < m' \leq 2$; and

b) adding said solution from step a) to at least one compound of a transition metal of Group 4 to 6 and

c) obtaining the solid catalyst component particles,

and adding a non-phthalic internal donor, at any step prior to step c).

The internal donor or precursor thereof is added preferably to the solution of step a).

According to the procedure above the Ziegler-Natta catalyst can be obtained via precipitation method or via emulsion (liquid/liquid two-phase system) - solidification method depending on the physical conditions, especially temperature used in steps b) and c).

In both methods (precipitation or emulsion-solidification) the catalyst chemistry is the same.

In precipitation method combination of the solution of step a) with at least one transition metal compound in step b) is carried out and the whole reaction mixture is kept at least at 50°C, more preferably in the temperature range of 55°C to 110°C, more preferably in the range of 70°C to 100°C, to secure full precipitation of the catalyst component in form of a solid particles (step c).

In emulsion - solidification method in step b) the solution of step a) is typically added to the at least one transition metal compound at a lower temperature, such as from -10 to below 50°C, preferably from -5 to 30°C. During agitation of the emulsion the temperature is typically kept at -10 to below 40°C, preferably from -5 to 30°C. Droplets of the dispersed phase of the emulsion form the active catalyst composition. Solidification (step c) of the droplets is suitably carried out by heating the emulsion to a temperature of 70 to 150°C, preferably to 80 to 110°C.

The catalyst prepared by emulsion - solidification method is preferably used in the present invention.

In a preferred embodiment in step a) the solution of $a_2$) or $a_3$) are used, i.e. a solution of (Ax') or a solution of a mixture of (Ax) and (Bx).

Preferably the Group 2 metal is magnesium.

The magnesium alkoxy compounds (Ax), (Ax') and (Bx) can be prepared in situ in the first step of the catalyst preparation process, step a), by reacting the magnesium compound with the alcohol(s) as described above, or said magnesium alkoxy compounds can be separately prepared magnesium alkoxy compounds or they can be even commercially available as ready magnesium alkoxy compounds and used as such in the catalyst preparation process of the invention.

Illustrative examples of alcohols (A) are monoethers of dihydric alcohols (glycol monoethers). Preferred alcohols (A) are $C_2$ to $C_4$ glycol monoethers, wherein the ether moieties comprise from 2 to 18 carbon atoms, preferably from 4 to 12 carbon atoms. Preferred examples are 2-(2-ethylhexyloxy)ethanol, 2-butyloxy ethanol, 2-hexyloxy ethanol and 1,3-propylene-glycol-monobutyl ether, 3-butoxy-2-propanol, with 2-(2-ethylhexyloxy)ethanol and 1,3-propyleneglycol-monobutyl ether, 3-butoxy-2-propanol being particularly preferred.

Illustrative monohydric alcohols (B) are of formula ROH, with R being straight-chain or branched $C_6$-$C_{10}$ alkyl residue. The most preferred monohydric alcohol is 2-ethyl-1-hexanol or octanol.

Preferably a mixture of Mg alkoxy compounds (Ax) and (Bx) or mixture of alcohols (A) and (B), respectively, are used and employed in a mole ratio of Bx:Ax or B:A from 8:1 to 2:1, more preferably 5:1 to 3:1.

Magnesium alkoxy compound may be a reaction product of alcohol(s), as defined above, and a magnesium compound selected from dialkyl magnesiums, alkyl magnesium alkoxides, magnesium dialkoxides, alkoxy magnesium halides and alkyl magnesium halides. Alkyl groups can be a similar or different $C_1$-$C_{20}$ alkyl, preferably $C_2$-$C_{10}$ alkyl. Typical alkyl-alkoxy magnesium compounds, when used, are ethyl magnesium butoxide, butyl magnesium pentoxide, octyl magnesium butoxide and octyl magnesium octoxide. Preferably the dialkyl magnesiums are used. Most preferred dialkyl magnesiums are butyl octyl magnesium or butyl ethyl magnesium.

It is also possible that magnesium compound can react in addition to the alcohol (A) and alcohol (B) also with a polyhydric alcohol (C) of formula $R''(OH)_m$ to obtain said magnesium alkoxide compounds. Preferred polyhydric alcohols, if used, are alcohols, wherein R" is a straight-chain, cyclic or branched $C_2$ to $C_{10}$ hydrocarbon residue, and m is an integer of 2 to 6.

The magnesium alkoxy compounds of step a) are thus selected from the group consisting of magnesium dialkoxides, diaryloxy magnesiums, alkyloxy magnesium halides, aryloxy magnesium halides, alkyl magnesium alkoxides, aryl magnesium alkoxides and alkyl magnesium aryloxides. In addition a mixture of magnesium dihalide and a magnesium dialkoxide can be used.

The solvents to be employed for the preparation of the present catalyst may be selected among aromatic and aliphatic straight chain, branched and cyclic hydrocarbons with 5 to 20 carbon atoms, more preferably 5 to 12 carbon atoms, or mixtures thereof. Suitable solvents include benzene, toluene, cumene, xylol, pentane, hexane, heptane, octane and nonane. Hexanes and pentanes are particular preferred.

Mg compound is typically provided as a 10 to 50 wt% solution in a solvent as indicated above. Typical commercially available Mg compound, especially dialkyl magnesium solutions are 20 - 40 wt% solutions in toluene or heptanes.

The reaction for the preparation of the magnesium alkoxy compound may be carried out at a temperature of 40° to 70°C. Most suitable temperature is selected depending on the Mg compound and alcohol(s) used.

The transition metal compound of Group 4 to 6 is preferably a titanium compound, most preferably a titanium halide, like TiCl4.

The non-phthalic internal donor used in the preparation of the catalyst used in the present invention is preferably selected from (di)esters of non-phthalic carboxylic (di)acids, 1,3-diethers, derivatives and mixtures thereof. Especially preferred donors are diesters of monounsaturated dicarboxylic acids, in particular esters belonging to a group comprising malonates, maleates, succinates, citraconates, glutarates, cyclohexene-1,2-dicarboxylates and benzoates, and any derivatives and/or mixtures thereof. Preferred examples are e.g. substituted maleates and citraconates, most preferably citraconates.

In emulsion method, the two phase liquid-liquid system may be formed by simple stirring and optionally adding (further) solvent(s) and additives, such as the turbulence minimizing agent (TMA) and/or the emulsifying agents and/or emulsion stabilizers, like surfactants, which are used in a manner known in the art for facilitating the formation of and/or stabilize the emulsion. Preferably, surfactants are acrylic or methacrylic polymers. Particular preferred are unbranched $C_{12}$ to $C_{20}$ (meth)acrylates such as poly(hexadecyl)-methacrylate and poly(octadecyl)-methacrylate and mixtures thereof. Turbulence minimizing agent (TMA), if used, is preferably selected from $\alpha$-olefin polymers of $\alpha$-olefin monomers with 6 to 20 carbon atoms, like polyoctene, polynonene, polydecene, polyundecene or polydodecene or mixtures thereof. Most preferable it is polydecene.

The solid particulate product obtained by precipitation or emulsion - solidification method may be washed at least once, preferably at least twice, most preferably at least three times with an aromatic and/or aliphatic hydrocarbons, preferably with toluene, heptane or pentane. The catalyst can further be dried, as by evaporation or flushing with nitrogen, or it can be slurried to an oily liquid without any drying step.

The finally obtained Ziegler-Natta catalyst is desirably in the form of particles having generally an average particle size range of 5 to 200 $\mu$m, preferably 10 to 100. Particles are compact with low porosity and have surface area below 20 g/m$^2$, more preferably below 10 g/m$^2$. Typically the amount of Ti is 1 to 6 wt%, Mg 10 to 20 wt% and donor 10 to 40 wt% of the catalyst composition.

Detailed description of preparation of catalysts is disclosed in WO 2012/007430, EP2610271, EP 261027 and EP2610272 which are incorporated here by reference.

The Ziegler-Natta catalyst (ZN-C) is preferably used in association with an alkyl aluminum cocatalyst (Co) and optionally external donors (ED).

As further component in the instant polymerization process an external donor (ED) is preferably present. Suitable external donors include certain silanes, ethers, esters, amines, ketones, heterocyclic compounds and blends of these. It is especially preferred to use a silane. It is most preferred to use silanes of the general formula

RapRbqSi(ORc)(4-p-q)

wherein Ra, Rb and Rc denote a hydrocarbon radical, in particular an alkyl or cycloalkyl group, and wherein p and q are numbers ranging from 0 to 3 with their sum p + q being equal to or less than 3. Ra, Rb and Rc can be chosen independently from one another and can be the same or different. Specific examples of such silanes are (tert-butyl)2Si(OCH3)2, (cyclohexyl)(methyl)Si(OCH3)2, (phenyl)2Si(OCH3)2 and (cyclopentyl)2Si(OCH3)2, or of general formula

Si(OCH2CH3)3(NR$^3$R$^4$)

wherein R$^3$ and R$^4$ can be the same or different a represent a hydrocarbon group having 1 to 12 carbon atoms.

R$^3$ and R$^4$ are independently selected from the group consisting of linear aliphatic hydrocarbon group having 1 to 12 carbon atoms, branched aliphatic hydrocarbon group having 1 to 12 carbon atoms and cyclic aliphatic hydrocarbon group having 1 to 12 carbon atoms. It is in particular preferred that R$^3$ and R$^4$ are independently selected from the group consisting of methyl, ethyl, n-propyl, n-butyl, octyl, decanyl, iso-propyl, iso-butyl, iso-pentyl, tert.-butyl, tert.-amyl, neopentyl, cyclopentyl, cyclohexyl, methylcyclopentyl and cycloheptyl.

More preferably both R$^3$ and R$^4$ are the same, yet more preferably both R$^3$ and R$^4$ are an ethyl group.

Especially preferred external donors (ED) are the dicyclopentyl dimethoxy silane donor (D-donor) or the cyclohexylmethyl dimethoxy silane donor (C-Donor).

In addition to the Ziegler-Natta catalyst (ZN-C) and the optional external donor (ED), a cocatalyst (Co) can be used. The co-catalyst (Co) is preferably a compound of group 13 of the periodic table (IUPAC), e.g. organo aluminum, such as an aluminum compound, like aluminum alkyl, aluminum halide or aluminum alkyl halide compound. Accordingly, in one specific embodiment the co-catalyst (Co) is a trialkylaluminium, like triethylaluminium (TEAL), dialkyl aluminium chloride or alkyl aluminium dichloride or mixtures thereof. In one specific embodiment the co-catalyst (Co) is triethylaluminium (TEAL).

Preferably the ratio between the co-catalyst (Co) and the external donor (ED) [Co/ED] and/or the ratio between the co-catalyst (Co) and the transition metal (TM) [Co/TM] should be carefully chosen.

Accordingly,

(a) the mol-ratio of co-catalyst (Co) to external donor (ED) [Co/ED] must be in the range of 5 to 45, preferably is in the range of 5 to 35, more preferably is in the range of 5 to 25; and optionally
(b) the mol-ratio of co-catalyst (Co) to titanium compound (TC) [Co/TC] must be in the range of above 80 to 500, preferably is in the range of 100 to 350, still more preferably is in the range of 120 to 300.

The heterophasic polypropylene composition according to this invention is preferably produced in the presence of

(a) a Ziegler-Natta catalyst (ZN-C) comprising an internal donor (ID),
(b) optionally a co-catalyst (Co), and
(c) optionally an external donor (ED).

As mentioned above the Ziegler-Natta catalyst (ZN-C) is optionally modified by the so called BNT-technology during the above described pre-polymerization step in order to introduce the polymeric nucleating agent.

Such a polymeric nucleating agent is as described above a vinyl polymer, such as a vinyl polymer derived from monomers of the formula

$$CH2 = CH-CHR1R2$$

as described above.

The weight ratio of vinyl compound to polymerization catalyst in the modification step of the polymerization catalyst preferably is 0.3 or more up to 40, such as 0.4 to 20 or more preferably 0.5 to 15, like 0.5 to 2.0.

The polymerization of the vinyl compound, e. g. VCH, can be done in any inert fluid that does not dissolve the polymer formed (e. g. polyVCH). It is important to make sure that the viscosity of the final catalyst/polymerized vinyl compound/inert fluid mixture is sufficiently high to prevent the catalyst particles from settling during storage and transport.

The adjustment of the viscosity of the mixture can be done either before or after the polymerization of the vinyl compound. It is, e. g., possible to carry out the polymerization in a low viscosity oil and after the polymerization of the vinyl compound the viscosity can be adjusted by addition of a highly viscous substance. Such highly viscous substance can be a "wax", such as an oil or a mixture of an oil with a solid or highly viscous substance (oil-grease). The viscosity of such a viscous substance is usually 1,000 to 15,000 cP at room temperature. The advantage of using wax is that the catalyst storing and feeding into the process is improved. Since no washing, drying, sieving and transferring are needed, the catalyst activity is maintained.

The weight ratio between the oil and the solid or highly viscous polymer is preferably less than 5: 1.

In addition to viscous substances, liquid hydrocarbons, such as isobutane, propane, pentane and hexane, can also be used as a medium in the modification step.

The polypropylenes produced with a catalyst modified with polymerized vinyl compounds contain essentially no free (unreacted) vinyl compounds. This means that the vinyl compounds shall be completely reacted in the catalyst modification step. To that end, the weight ratio of the (added) vinyl compound to the catalyst should be in the range of 0.05 to 10, preferably less than 3, more preferably about 0.1 to 2.0, and in particular about 0.1 to 1.5. It should be noted that no benefits are achieved by using vinyl compounds in excess.

Further, the reaction time of the catalyst modification by polymerization of a vinyl compound should be sufficient to allow for complete reaction of the vinyl monomer, i.e. the polymerization is continued until the amount of unreacted vinyl compounds in the reaction mixture (including the polymerization medium and the reactants) is less than 0.5 wt%, in particular less than 2000 ppm by weight (shown by analysis). Thus, when the pre-polymerized catalyst contains a maximum of about 0.1 wt% vinyl compound, the final vinyl compound content in the polypropylene will be below the limit of determination using the GC-MS method (< 0.01 ppm by weight). Generally, when operating on an industrial scale, a polymerization time of at least 30 minutes is required, preferably the polymerization time is at least 1 hour and in particular at least 5 hours. Polymerization times even in the range of 6 to 50 hours can be used. The modification can be done at temperatures of 10 to 60°C, preferably 15 to 55°C.

General conditions for the modification of the catalyst are also disclosed in WO 00/6831, incorporated herein by reference with respect to the modification of the polymerization catalyst. The preferred embodiments as described previously in the present application with respect to the vinyl compound also apply with respect to the polymerization catalyst of the present invention and the preferred polypropylene composition in accordance with the present invention. Suitable media for the modification step include, in addition to oils, also aliphatic inert organic solvents with low viscosity, such as pentane and heptane. Furthermore, small amounts of hydrogen can be used during the modification.

**Component (B) styrene-based elastomer**

The additional required component in the present invention is a styrene-based elastomer.
It has been discovered that not any styrene-based elastomer is suitable for the present invention but only a specific class of such elastomers. Accordingly for the present invention a styrene-based elastomer being an unsaturated styrene block copolymer containing at least one diene comonomer, preferably selected from butadiene and isoprene and having a styrene content of 5.0 to 50.0 wt% measured by Fourier transform infrared spectroscopy (FTIR).

Accordingly it is appreciated that the styrene-based elastomer (B), preferably a styrene-isoprene-styrene (SIS) block copolymer, a styrene-butadiene-styrene (SBS) block copolymer or a styrene-isoprene-butadiene-styrene (SIBS) block copolymer, more preferably a styrene-isoprene-styrene (SIS) block copolymer, has a styrene content of at least 5.0 wt%, preferably at least 10.0 wt% and more preferably at least 15.0 wt%. The upper limit is 50.0 wt%, preferably 40.0 wt%, more preferably 35.0 wt% and even more preferably 30.0 wt%.

Further it is appreciated that the styrene-based elastomer (B), preferably a styrene-isoprene-styrene (SIS) block copolymer has a melt flow rate $MFR_2$ (230°C, ISO 1133) of not more than 200.0 g/10min, preferably not more than 150.0 g/10min and more preferably not more than 100.0 g/10min.
On the other hand the melt flow rate of the styrene-based elastomer (B), preferably a styrene-isoprene-styrene (SIS) block copolymer or a styrene-butadiene-styrene block copolymer, more preferably a styrene-isoprene-styrene (SIS) block copolymer, shall not fall below 2.0 g/10min. Accordingly, a preferred range is from 2.0 to 200.0 g/10min, more preferred of 8.0 to 150.0 g/10min and even more preferred from 10.0 to 100.0 g/10min.

Further the styrene-based elastomer (B), preferably a styrene-isoprene-styrene (SIS) block copolymer may be defined by its density.
Thus it is appreciated that the styrene-based elastomer (B), preferably a styrene-isoprene-styrene (SIS) block copolymer, has a density of from 0.900 g/cm$^3$ to 0.950 g/cm$^3$, preferably from 0.910 g/cm$^3$ to 0.950 g/cm$^3$ and more preferably from 0.915 to 0.945 g/cm$^3$.

Additionally or alternatively the styrene-based elastomer (B), preferably a styrene-isoprene-styrene (SIS) block copolymer can be defined by the Shore A hardness. Thus it is appreciated that the styrene-based elastomer (B) has a Shore A hardness measured according to ASTM D 2240 from 25 to 80, preferably from 30 to 75.

Examples of styrene-based elastomer (B) are the DEXCO TSRC - product line VECTOR for SIS and SBS, commercialised as VECTOR 4111A (SIS, Shore A hardness of 40, styrene content of 18wt%, density 0.93 g/cm$^3$), VECTOR 4211 (SIS, 29 wt% styrene, density 0.94 g/cm$^3$, Shore A 68) or VECTOR 8508 (SBS, 29 wt% styrene, density 0.94 g/cm$^3$, Shore A 67); or products from the Kraton D(SBS), Kraton D(SIS) or Kraton S(SIBS) product lines, like e.g. Kraton D1102 A (SBS, 29.5 wt% styrene, density 0.94 g/cm$^3$, Shore A 70), Kraton D1111 K (SIS, 22 wt% styrene, density 0.93 g/cm$^3$, Shore A 45) or Kraton D1119 B (SIS, 16 wt% styrene, density 0.94 g/cm$^3$, Shore A 38) or from the Versalis Eni - product line "Europrene" for SIS and SBS, examples Europrene SOL T 190 (SIS, 16 wt% styrene, density 0.92 g/cm$^3$, Shore A 30) or Europrene SOL T 6205 (SBS, 25 wt% styrene, density 0.93 g/cm$^3$, Shore A 68);

**Ad) grafted heterophasic propylene copolymer composition**

The composition of the present invention comprises component (A) and component (B) as described above, whereby component (B) is grafted onto component (A).

The amount of component (A), the base heterophasic propylene copolymer, is present in an amount of from 30.0 to 95.0 wt%, preferably from 40.0 to 90.0 wt% more preferably from 50.0 to 85.0 wt% and even more preferably from 55.0 to 80.0 wt%.

Thus the amount of component (B), the styrene-based elastomer, is present in an amount of from 5.0 to 70.0 wt%, preferably from 10.0 to 60.0 wt%, more preferably from 15.0 to 50.0 wt% and even more preferably from 20.0 to 45.0 wt%.

The grafted heterophasic propylene copolymer composition is characterized by a composition melt flow rate ($MFR_C$) (ISO 1133; 230°C; 2.16kg) in the range of 0.5 to 50.0 g/10 min, preferably in the range of 1.0 to 40.0 g/10 min, more preferably in the range of 1.5 to 30.0 g/10 min and even more preferably in the range of 2.0 to 15.0 g/10 min.

The grafted heterophasic propylene copolymer composition is further and/or alternatively defined by the gel content. The gel content is a good indicator for the chemical modification of the inventive composition (indication of unwanted crosslinking).

It is preferred that the gel content is kept at a rather low degree, meaning that the in the process of preparing the inventive composition, the predominant reaction is grafting not crosslinking. Accordingly the present invention is featured by a gel content in the range of 0.05 wt% to less than 5.00 wt% determined as the relative amount of polymer insoluble in boiling xylene (xylene hot insoluble fraction, XHI fraction). Preferably the gel content of the inventive composition is in the range 0.10 wt% to 2.00 wt% and more preferably in the range of 0.15 wt% to 1.50 wt%

Moreover the grafted heterophasic propylene copolymer composition of the instant invention has preferably a tensile modulus measured according to ISO 527-1,2 (cross head speed = 1 mm/min) of below 350 MPa, preferably below 320 MPa and more preferably below 300 MPa. The lower limit may be 100 MPa, preferable 200 MPa.

Additionally it is preferred that the grafted heterophasic propylene copolymer composition of the present invention has a rather high impact strength, especially at low temperatures. Accordingly the inventive composition has an impact strength measured according to the Charpy notched impact test according to ISO 179 (1eA) at -20°C of at least 15.0 kJ/m$^2$, preferably of at least 30.0 kJ/m$^2$, more preferably of at least 50.0 kJ/m$^2$ and even more preferably of at least 65 kJ/m$^2$.

The upper limit of Charpy notched impact test according to ISO 179 (1eA) at -20°C may be 150 kJ/m$^2$, preferably 100 kJ/m$^2$.

Furthermore the inventive composition has an impact strength measured according to the Charpy notched impact test according to ISO 179 (1eA) at -30°C of at least 7.0 kJ/m$^2$, preferably of at least 10.0 kJ/m$^2$, more preferably of at least 12.0 kJ/m$^2$ and even more preferably of at least 14 kJ/m$^2$.

The upper limit of Charpy notched impact test according to ISO 179 (1eA) at -30°C may be 60 kJ/m$^2$, preferably 50 kJ/m$^2$.

The grafted heterophasic propylene copolymer composition of the present invention has a F30 melt strength of at least 3.0 cN, preferably of at least 4.5 cN and more preferably at least 5.0 cN, wherein the F30 melt strength are measured with the Rheotens test at 200°C according to ISO 16790:2005.

*Preparation of the grafted heterophasic propylene copolymer composition*

The grafted heterophasic propylene copolymer composition of the present invention can be prepared by a simple grafting process, which can also be named reactive melt modification process.

The selection of these two specific polymers (A) and (B) guarantees good compatibility of the two components in view of an efficient and homogeneous grafting of polymer (B) onto polymer (A).

Thus according to a further embodiment the present invention is related to a process for producing the grafted heterophasic propylene copolymer composition, such process being a reactive melt modification process comprising the steps of

> i) mixing component (A), the heterophasic polypropylene copolymer, with component (B), the styrene-based elastomer and a thermally decomposing free radical-forming compound (C) and
> ii) extruding said mixture.

In this process a thermally decomposing free radical-forming compound (C) is added to initiate the grafting reaction of styrene-based elastomer (B) onto the base heterophasic propylene copolymer (A).

Peroxides are preferred thermally decomposing free radical-forming agents.

The following listed peroxides are in particular preferred:

Acyl peroxides: benzoyl peroxide, 4-chlorobenzoyl peroxide, 3-methoxybenzoyl peroxide and/or methyl benzoyl peroxide.

Alkyl peroxides: allyl t-butyl peroxide, 2,2-bis(t-butylperoxybutane), 1,1-bis(t-butylperoxy)-3,3,5-trimethylcyclohexane, n-butyl-4,4-bis(t-butylperoxy) valerate, diisopropylaminomethyl-t-amyl peroxide, dimethylaminomethyl-tamyl peroxide, diethylaminomethyl-t-butyl peroxide, dimethylaminomethyl-t-butyl peroxide, 1,1-di-(t-amylperoxy)cyclohexane, t-amyl peroxide, t-butylcumyl peroxide, t-butyl peroxide and/or 1-hydroxybutyl n-butyl peroxide.

Peresters and peroxy carbonates: butyl peracetate, cumyl peracetate, cumyl perpropionate, cyclohexyl peracetate, di-t-butyl peradipate, di-t-butyl perazelate, di-t-butyl perglutarate, di-t-butyl perthalate, di-t-butyl persebacate, 4-nitrocumyl perpropionate, 1-phenylethyl perbenzoate, phenylethyl nitro-perbenzoate, t-butylbicyclo-(2,2,1)heptane percarboxylate, t-butyl-4-carbomethoxy perbutyrate, t-butylcyclobutane percarboxylate, t-butylcyclohexyl peroxycarboxylate, t-butylcyclopentyl percarboxylate, t-butylcyclopropane percarboxylate, t-butyldimethyl percinnamate, t-butyl-2-(2,2- diphenylvinyl) perbenzoate, t-butyl-4-methoxy perbenzoate, t-butylperbenzoate, t-butylcarboxycyclohexane, t-butyl pernaphthoate, t-butyl peroxyisopropylcarbonate, t-butyl pertoluate, t-butyl-1-phenylcyclopropyl percarboxylate, t-butyl-2-propylperpentene-2-oate, t-butyl-1-methylcyclopropyl percarboxylate, t-butyl-4-nitrophenyl peracetate, t-butylnitrophenyl peroxycarbamate, t-butyl-N-succiimido percarboxylate, t-butyl percrotonate, t-butyl permaleic acid, t-butyl permethacrylate, t-butyl peroctoate, t-butyl peroxyisopropylcarbonate, t-butyl perisobutyrate, t-butyl peracrylate and/or t-butyl perpropionate;

Mixtures of one or more of these above listed free radical-forming agents can also be used.

Compound (C), preferably one or more of the above listed peroxides, is present at a concentration of at 0.3 to 4.0 wt% relative to the total amount of polymers A and B, typically between 0.5 and 3.0 wt% relative to the total amount of polymers A and B.

Accordingly, the grafted, impact modified soft heterophasic propylene copolymer composition in accordance with the present invention may be prepared by compounding the components within suitable melt mixing devices for preparing polymeric compounds, including in particular extruders like single screw extruders as well as twin screw extruders. Especially preferred are twin screw extruders including high intensity mixing and kneading sections. Suitable melt temperatures, i.e. average barrel temperatures, for preparing the compositions are in the range from 170 to 280 °C, preferably in the range from 180 to 250 °C.

The grafting or reactive melt modification can be for instance effected by dosing the polymer components (A) and (B) separately into a twin screw extruder like Prism TSE24 40D with a temperature profile like 80/200/210/220/220/230/230/220/225/220 °C and a screw speed of 200 to 500 rpm, preferably 250 to 400 rpm, more preferably around 300 rpm (e.g. 280 to 320 rpm). After heating and melting of the polymer mixture, a solution of the thermally decomposing free radical-forming agent, like tert-butylperoxy isopropyl carbonate, preferably in acetone (10.0 wt%) is injected directly into the extruder.

The polymer melt / liquid /gas mixture is passed through the extruder, then to intensive devolatilisation, discharged and pelletised.

## Use

The invention is also directed to the use of the inventive heterophasic propylene copolymer composition. Accordingly the present invention is directed to the use of the use of the grafted heterophasic propylene copolymer composition for producing articles, like extruded films or tubes, blow moulded or injection moulded articles, and to the articles comprising the grafted heterophasic propylene copolymer composition, preferably comprising more than 90.0 wt% of the grafted, impact modified heterophasic propylene copolymer composition.

Especially such articles are medical tubes or pouches comprising more than 90.0 wt% of the grafted heterophasic propylene copolymer composition.

The invention will now be illustrated by reference to the following non-limiting examples

## Methods

**The xylene soluble fraction at room temperature (XCS, wt%):** The amount of the polymer soluble in xylene is determined at 25 °C according to ISO 16152; 5th edition; 2005-07-01.

## Intrinsic viscosity (IV)

The intrinsic viscosity (IV) value increases with the molecular weight of a polymer. The IV values e.g. of the XCS were measured according to ISO 1628/1 in decalin at 135°C.

**The glass transition temperature Tg** is determined by dynamic mechanical thermal analysis according to ISO 6721-7. The measurements are done in torsion mode on compression moulded samples (40x10x1 mm3) between -100°C and +150°C with a heating rate of 2 °C/min and a frequency of 1 Hz.

**MFR2 (230°C)** is measured according to ISO 1133 (230°C, 2.16 kg load)

The melt flow rate is measured as the MFR2 in accordance with ISO 1133 15 (230°C, 2.16 kg load) for polypropylene and in accordance with ISO 1133 (190°C, 2.16 kg load) for polyethylene and is indicated in g/10 min. The MFR is an indication of the flowability, and hence the processability, of the polymer. The higher the melt flow rate, the lower the viscosity of the polymer.

The $MFR_2$ of a fraction (B) produced in the presence of a fraction (A) is calculated using the measured values of MFR2 of fraction (A) and the mixture received after producing fraction (B) ("final"):

$$Log\big(MFR_{final}\big) = weight\ fraction(A) * Log(MFR_A) + weight\ fraction(B) * Log(MFR_B)$$

## Comonomer determination by NMR spectroscopy

Quantitative nuclear-magnetic resonance (NMR) spectroscopy was further used to quantify the comonomer content and comonomer sequence distribution of the polymers. Quantitative $^{13}C\{^1H\}$ NMR spectra were recorded in the solution-state using a Bruker Advance III 400 NMR spectrometer operating at 400.15 and 100.62 MHz for $^1H$ and $^{13}C$

respectively. All spectra were recorded using a $^{13}$C optimised 10 mm extended temperature probehead at 125°C using nitrogen gas for all pneumatics. Approximately 200 mg of material was dissolved in 3 ml of 1,2-tetrachloroethane-$d_2$ (TCE-$d_2$) along with chromium-(III)-acetylacetonate (Cr(acac)$_3$) resulting in a 65 mM solution of relaxation agent in solvent (Singh, G., Kothari, A., Gupta, V., Polymer Testing 28 5 (2009), 475). To ensure a homogenous solution, after initial sample preparation in a heat block, the NMR tube was further heated in a rotatary oven for at least 1 hour. Upon insertion into the magnet the tube was spun at 10 Hz. This setup was chosen primarily for the high resolution and quantitatively needed for accurate ethylene content quantification. Standard single-pulse excitation was employed without NOE, using an optimised tip angle, 1 s recycle delay and a bi-level WALTZ16 decoupling scheme (Zhou, Z., Kuemmerle, R., Qiu, X., Redwine, D., Cong, R., Taha, A., Baugh, D. Winniford, B., J. Mag. Reson. 187 (2007) 225; Busico, V., Carbonniere, P., Cipullo, R., Pellecchia, R., Severn, J., Talarico, G., Macromol. Rapid Commun. 2007, 28, 1128). A total of 6144 (6k) transients were acquired per spectra. Quantitative $^{13}$C{$^1$H} NMR spectra were processed, integrated and relevant quantitative properties determined from the integrals using proprietary computer programs. All chemical shifts were indirectly referenced to the central methylene group of the ethylene block (EEE) at 30.00 ppm using the chemical shift of the solvent. This approach allowed comparable referencing even when this structural unit was not present. Characteristic signals corresponding to the incorporation of ethylene were observed Cheng, H. N., Macromolecules 17 (1984), 1950).

With characteristic signals corresponding to 2,1 erythro regio defects observed (as described in L. Resconi, L. Cavallo, A. Fait, F. Piemontesi, Chem. Rev. 2000, 100 (4), 1253, in Cheng, H. N., Macromolecules 1984, 17, 1950, and in W-J. Wang and S. Zhu, Macromolecules 2000, 33 1157) the correction for the influence of the regio defects on determined properties was required. Characteristic signals corresponding to other types of regio defects were not observed.

The comonomer fraction was quantified using the method of Wang et. al. (Wang, W-J., Zhu, S., Macromolecules 33 (2000), 1157) through integration of multiple signals across the whole spectral region in the $^{13}$C{$^1$H} spectra. This method was chosen for its robust nature and ability to account for the presence of regio-defects when needed. Integral regions were slightly adjusted to increase applicability across the whole range of encountered comonomer contents. For systems where only isolated ethylene in PPEPP sequences was observed the method of Wang et. al. was modified to reduce the influence of non-zero integrals of sites that are known to not be present. This approach reduced the overestimation of ethylene content for such systems and was achieved by reduction of the number of sites used to determine the absolute ethylene content to:

$$E = 0.5(S\beta\beta + S\beta\gamma + S\beta\delta + 0.5(S\alpha\beta + S\alpha\gamma))$$

Through the use of this set of sites the corresponding integral equation becomes:

$$E = 0.5(I_H + I_G + 0.5(I_C + I_D))$$

using the same notation used in the article of Wang et. al. (Wang, W-J., Zhu, S., Macromolecules 33 (2000), 1157). Equations used for absolute propylene content were not modified.

The mole percent comonomer incorporation was calculated from the mole fraction:

$$E\ [mol\%] = 100 * fE$$

The weight percent comonomer incorporation was calculated from the mole fraction:

$$E\ [wt\%] = 100 * (fE * 28.06) / ((fE * 28.06) + ((1-fE) * 42.08))$$

The comonomer sequence distribution at the triad level was determined using the analysis method of Kakugo et al. (Kakugo, M., Naito, Y., Mizunuma, K., Miyatake, T. Macromolecules 15 (1982) 1150). This method was chosen for its robust nature and integration regions slightly adjusted to increase applicability to a wider range of comonomer contents.

**Melting Temperature**

Melting temperature (Tm) was measured with a Mettler TA820 differential scanning calorimetry (DSC) apparatus on 5 to 10 mg samples. DSC was performed according to ISO 3146 (part 3, method C2) in a heat/cool/heat 15 cycle with a scan rate of 10 °C/min in the temperature range of +23 to +210°C. Melting temperature was determined from the second heating step.

**Tensile modulus** in machine direction was determined according to ISO 527-1,2 at 23°C on 50 $\mu$m cast film produced on a monolayer cast film line with a melt temperature of 220°C and a chill roll temperature of 20°C. Testing was performed at a cross head speed of 1 mm/min.

### Styrene content

The styrene content is measured by Fourier transform infrared spectroscopy (FTIR). A thin film of 300 mm thickness is prepared from pelletized material by hot-pressing (190 °C, 100 bar, 1 minute). Per sample, two films are prepared. The so prepared film-samples are measured by a Perkin Elmer IR-Spectrophotometer System 2000FTIR.

The peak at 1602 cm$^{-1}$ (Phenyl-Absorption) is integrated and evaluated by using an internally established calibration curve. The arithmetic mean of two measurements is given as result. Calibration: Various polypropylene-compounds consisting of PP and a styrene-containing elastomer (of known styrene-content) are prepared and measured according to the method described above.

### Charpy notched impact strength

The Charpy notched impact strength (NIS) was measured according to ISO 179 1eA at +23 °C and -20 °C, using injection moulded bar test specimens of 80x10x4 mm$^3$ prepared in accordance with EN ISO 1873-22.

**Tensile modulus** in machine direction was determined according to ISO 527-3 at 23°C on 50 $\mu$m cast film produced on a monolayer cast film line with a melt temperature of 220°C and a chill roll temperature of 20°C. Testing was performed at a cross head speed of 1 mm/min.

### F30 - Rheotens test

The test described herein follows ISO 16790:2005.

The strain hardening behaviour is determined by the method as described in the article "Rheotens-Mastercurves and Drawability of Polymer Melts", M. H. Wagner, Polymer Engineering and Sience, Vol. 36, pages 925 to 935. The content of the document is included by reference. The strain hardening behaviour of polymers is analysed by Rheotens apparatus (product of Gottfert, Siemensstr.2, 74711 Buchen, Germany) in which a melt strand is elongated by drawing down with a defined acceleration.

The Rheotens experiment simulates industrial spinning and extrusion processes. In principle a melt is pressed or extruded through a round die and the resulting strand is hauled off. The stress on the extrudate is recorded, as a function of melt properties and measuring parameters (especially the ratio between output and haul-off speed, practically a measure for the extension rate). For the results presented below, the materials were extruded with a lab extruder HAAKE Polylab system and a gear pump with cylindrical die (L/D = 6.0/2.0 mm). The gear pump was pre-adjusted to a strand extrusion rate of 5 mm/s, and the melt temperature was set to 200°C. The spinline length between die and Rheotens wheels was 80 mm. At the beginning of the experiment, the take-up speed of the Rheotens wheels was adjusted to the velocity of the extruded polymer strand (tensile force zero): Then the experiment was started by slowly increasing the take-up speed of the Rheotens wheels until the polymer filament breaks. The acceleration of the wheels was small enough so that the tensile force was measured under quasi-steady conditions. The acceleration of the melt strand (2) drawn down is 120 mm/sec2. The Rheotens was operated in combination with the PC program EXTENS. This is a real-time data-acquisition program, which displays and stores the measured data of tensile force and drawdown speed. The end points of the Rheotens curve (force versus pulley rotary speed) are taken as the melt strength and drawability values.

### Inventive Examples

The solid catalyst component was prepared otherwise according to Example 8 of WO 2004/029112, except that diethylaluminium chloride was used as an aluminium compound instead of triethylaluminium.

The thus obtained catalyst was used along with triethyl-aluminium (TEAL) as co-catalyst and dicyclopentyl dimethoxy silane (D-Donor) as donor for preparing the heterophasic polypropylene copolymer (A) used according to the present invention. Polymerization was done in Borstar plant with a pre-polymerization step, one loop reactor and 2 gas-phase reactors. The conditions can be seen in Table 1. The matrix was a random propylene copolymer, thus a RAHECO was produced.

**Table 1: Preparation of heterophasic polypropylene copolymer (A) with random copolymer as matrix, i.e. RAHECO**

| | | RAHECO |
|---|---|---|
| **Prepolymerization** | | |
| TEAL/Ti | [mol/mol] | 210 |
| TEAL/donor | [mol/mol] | 6.95 |
| Temperature | [°C] | 20 |
| res.time | [h] | 0.38 |
| **Loop** | | |
| Temperature | [°C] | 70 |
| Pressure | [kPa] | 5000 |
| Split | [%] | 35 |
| H2/C3 ratio | [mol/kmol] | 1.7 |
| C2/C3 ratio | [mol/kmol] | 4.5 |
| $MFR_2$ | [g/10min] | 8.0 |
| XCS | [wt%] | 6.5 |
| C2 content | [mol%] | 3.7 |
| **GPR 1** | | |
| Temperature | [°C] | 80 |
| Pressure | [kPa] | 2800 |
| Split | [%] | 52 |
| H2/C3 ratio | [mol/kmol] | 21.5 |
| C2/C3 ratio | [mol/kmol] | 28.1 |
| $MFR_2(MFR_M)$ | [g/10min] | 8.0 |
| XCS | [wt%] | 7.5 |
| C2 content | [mol%] | 5.9 |
| **GPR 2** | | |
| Temperature | [°C] | 75 |
| Pressure | [kPa] | 2600 |
| Split | [%] | 13 |
| C2/C3 ratio | [mol/kmol] | 490 |
| H2/C2 ratio | [mol/kmol] | 530 |
| $MFR_2(MFR_T)$ | [g/10min] | 7.0 |
| XCS | [wt%] | 20.0 |
| IV (XCS) | [dl/g] | 1.5 |
| C2 (XCS) | [mol%] | 40.3 |

(continued)

| GPR 2 | | |
|---|---|---|
| C2 content | [mol%] | 11.5 |

| C2 ethylene |
| H2/C3 ratio hydrogen / propylene ratio |
| C2/C3 ratio ethylene / propylene ratio |
| H2/C2 ratio hydrogen / ethylene ratio |
| GPR 1/2 1st/2nd gas phase reactor |
| Loop Loop reactor |

The RAHECO was mixed in a twin-screw extruder with 0.2 wt.-% of Irganox B225 (1:1-blend of Irganox 1010 (Pentaerythrityl-tetrakis(3-(3',5'-di-tert.butyl-4-hydroxytoluyl)-propionate and tris (2,4-di-t-butylphenyl) phosphate) phosphite) of BASF AG, Germany) and 0.1 wt% calcium stearate.

In a next step the RAHECO (A) was mixed with 35.0 wt% of styrene-based elastomer (B). The styrene-based elastomer (B) was VECTOR 4111A, a styrene-isoprene-styrene (SIS) block copolymer provided by DEXCO.

This styrene-based elastomer (B) has according to DEXCO a styrene content of 18.0 wt%, a $MFR_5$ 200°C of 12.0 g/10 min and a Shore A (ASTM D2240) of 40, density of 0.93 g/cm$^3$.

Mixing was done in a co-rotating twin screw extruder (Prism TSE24 40D) with a temperature profile 80/200/210/220/220/230/230/220/225/220°C and a screw speed of 300 rpm. After heating and melting of the polymer mixture (i.e. components (A) and (B)), a solution of the thermally decomposing free radical-forming agent, i.e. tert-butylperoxy isopropyl carbonate, in acetone, provided by AkzoNobel was injected directly into the extruder.

The polymer melt / liquid /gas mixture is passed through the extruder.

Afterwards the polymer melt mixture was discharged and pelletized and grafted, impact modified RAHECO compositions were yielded. The properties of these compositions can be seen in Table 2.

CE1 is the pure base RAHECO produced according to Table 1.

CE2 is a melt blend of the pure base RAHECO produced according to Table 1 and 35.0 wt% of styrene-based elastomer (B), being again VECTOR 4111A, without addition of POX.

**Table 2:**

| | unit | CE1 | CE2 | IE1 | IE2 | IE3 |
|---|---|---|---|---|---|---|
| RAHECO (A) | Wt% | 100 | 65 | 64.5 | 63.5 | 62.5 |
| VECTOR 4111A | Wt% | 0 | 35 | 35 | 35 | 35 |
| POX | Wt% | 0 | 0 | 0.5 | 1.5 | 2.5 |
| $MFR_c$ | g/10min | 8.0 | 9.5 | 2.8 | 2.4 | 8.7 |
| $XCS_c$ | Wt% | 20.0 | 46.8 | 41.0 | 20.8 | 14.4 |
| XHI (gels) | Wt% | 0 | 0 | 0.45 | 1.05 | 0.16 |
| F30 | cN | n.m. | 2.6 | 5.1 | 5.2 | 5.2 |
| Tensile Modulus | MPa | 650 | 366 | 284 | 285 | 287 |
| NIS -20°C | kJ/m$^2$ | 1.0 | 11.3 | 70.8 | 71.3 | 71.6 |
| NIS -30°C | kJ/m$^2$ | 2.2 | 5.1 | 30.0 | n.m. | 14.8 |
| $MFR_c$ $MFR_2$ of composition $XCS_c$ XCS of composition | | | | | | |

## Claims

1. Grafted heterophasic propylene copolymer composition, which comprises

(A) 30.0 to 95.0 wt% of a heterophasic polypropylene copolymer having a melt flow rate (MFR 230°C/2.16kg, ISO1133) of from 0.1 to 100 g/10min and comprising

(a1) a matrix (M) being a crystalline polypropylene homo- or copolymer and
(a2) and elastomeric copolymer (E) dispersed in said matrix (M)
and

(B) 5.0 to 70.0 wt% of a styrene-based elastomer being a unsaturated styrene block copolymer containing at least one diene comonomer and having a styrene content of from 5.0 to 50.0 wt% measured by Fourier transform infrared spectroscopy (FTIR),
and wherein the styrene-based elastomer (B) is grafted onto the base heterophasic polypropylene copolymer (A), wherein the grafted heterophasic propylene copolymer composition has an impact strength measured according to the Charpy notched impact test according to ISO 179 (1eA) at -20°C of at least 15.0 kJ/m$^2$, impact strength measured according to the Charpy notched impact test according to ISO 179 (1eA) at -30°C of at least 7.0 kJ/m$^2$ and F30 melt strength of at least 3.0 cN measured with the Rheotens test at 200°C according to ISO 16790:2005.

2. The heterophasic propylene copolymer composition according to claim 1, wherein the heterophasic polypropylene copolymer has xylene cold soluble (XCS) content determined according to ISO 16152 (25°C) in the range of from 10.0 to 50.0 wt%, the xylene cold soluble (XCS) content having an intrinsic viscosity (iV) determined according to DIN ISO 1628/1 (in decalin at 135°C) in the range of from 1.0 to 5.0 dl/g.

3. The heterophasic propylene copolymer composition according to claim 1 or 2, wherein the heterophasic polypropylene copolymer has

• a total melt flow rate (MFRT) (ISO 1133; 230°C; 2.16kg) in the range of from 0.1 to 100.0 g/10 min,
• a total ethylene comonomer content, in polymerized form, in the range of from 2.5 to 15.0 wt% ,
• a first glass transition temperature Tg(1) in the range of from -12°C to +4°C and/or a second glass transition temperature Tg(2) in the range of from -65°C to -30°C.

4. The heterophasic propylene copolymer composition according to any one of the preceding claims, wherein the matrix (M) of the heterophasic polypropylene copolymer is a random propylene- copolymer (R-PP) wherein the random propylene copolymer (R-PP) has

• a melt flow rate MFR$_2$ (230°C) measured according to ISO 1133 in the range of from 0.5 to 100.0 g/10min, and
• a ethylene and/or C$_4$ to C$_8$ $\alpha$-olefins comonomer content, in polymerized form, in the range of from 1.0 to 12.0 wt%.

5. The heterophasic propylene copolymer composition any one of the preceding claims, wherein the random propylene copolymer (R-PP) and the elastomeric copolymer (E) both being made from ethylene and propylene monomers only.

6. The heterophasic propylene copolymer composition according to any one of the preceding claims, wherein the diene comonomer of the styrene based elastomer (B) is selected from butadiene and isoprene.

7. The heterophasic propylene copolymer composition according to any one of the preceding claims, wherein the styrene based elastomer (B) has a

• melt flow rate MFR$_2$ (230 °C, ISO 1133) in the range of from 2.0 to 200.0 g/10min and
• a Shore A hardness measured according to ASTM D 2240 in the range of from 25 to 65.

8. The heterophasic propylene copolymer composition according to any one of the preceding claims, wherein the heterophasic propylene copolymer composition has

• a melt flow rate MFR$_2$ (230 °C, ISO 1133) in the range of from 0.5 to 50.0 g/10min and
• a xylene hot insoluble content in the range of from 0.10 to 2.00 wt%.

9. The heterophasic propylene copolymer composition according to any one of the preceding claims, wherein the heterophasic propylene copolymer composition has

• a tensile modulus according to ISO 527-1,2 measured on a 50$\mu$m cast film of below 350 MPa and
• a Charpy notched impact strength according to ISO 179 (1eA) at -20°C of at least 15.0 kJ/m$^2$, and at -30°C of at least 7.0 kJ/m$^2$.

10. Process for producing the grafted heterophasic propylene copolymer composition according to any one of claims 1 to 9, which process is a reactive melt modification process comprising the steps of

i) mixing component (A), the heterophasic polypropylene copolymer with component (B), the styrene-based elastomer and a thermally decomposing free radical-forming compound (C) and
ii) extruding said mixture.

11. Process according to claim 10, wherein the heterophasic polypropylene copolymer (A) is produced in a multistage process comprising at least two reactors connected in series.

12. Process according to claim 10 or 11, wherein the thermally decomposing free radical-forming compound (C) is a peroxide.

13. Use of the grafted heterophasic polypropylene copolymer composition according to any one of claims 1 to 9 for producing articles selected from extruded films or tubes, pouches, blow moulded or injection moulded articles.

14. Articles comprising more than 90 wt% of the grafted heterophasic polypropylene copolymer composition according to any one of claims 1 to 9,

15. Articles according to claim 14, wherein the articles are medical tubes or pouches comprising more than 90 wt% of the grafted heterophasic polypropylene copolymer composition according to any one of claims 1 to 9.

**Patentansprüche**

1. Gepfropfte heterophasische Propylen-Copolymer-Zusammensetzung, die umfasst

(A) 30,0 bis 95,0 Gew.-% eines heterophasischen Polypropylen-Copolymers mit einer Schmelzflußrate (MFR 230°C/2,16kg, ISO1133) von 0,1 bis 100 g/10min und umfassend

(a1) eine Matrix (M), die ein kristallines Polypropylen-Homo- oder -Copolymer ist, und
(a2) und elastomeres Copolymer (E), dispergiert in der Matrix (M) und

(B) 5,0 bis 70,0 Gew.-% eines Elastomers auf Styrolbasis, das ein ungesättigtes Styrolblock-Copolymer ist, das mindestens ein Dien-Comonomer enthält und einen Styrolgehalt von 5,0 bis 50,0 Gew.-% hat, gemessen durch Fourier-Transformations-Infrarotspektroskopie (FTIR),
und wobei das Elastomer auf Styrolbasis (B) auf das heterophasische Polypropylen-Basiscopolymer (A) aufgepfropft ist,
wobei die gepfropfte heterophasische Propylencopolymer-Zusammensetzung eine Schlagzähigkeit, gemessen gemäß dem Charpy-Kerbschlagtest gemäß ISO 179 (1eA) bei -20°C, von mindestens 15,0 kJ/m2, eine Schlagzähigkeit, gemessen gemäß dem Charpy-Kerbschlagtest gemäß ISO 179 (1eA) bei -30°C, von mindestens 7,0 kJ/m2 und eine F30-Schmelzfestigkeit von mindestens 3,0 cN, gemessen mit dem Rheotens-Test bei 200°C gemäß ISO 16790:2005, aufweist.

2. Heterophasische Propylen-Copolymer-Zusammensetzung nach Anspruch 1, wobei das heterophasische Polypropylen-Copolymer einen in kaltem Xylol löslichen Gehalt (XCS), bestimmt nach ISO 16152 (25°C), im Bereich von 10,0 bis 50,0 Gew.-% aufweist, wobei der in kaltem Xylol lösliche Gehalt (XCS) eine intrinsische Viskosität (IV), bestimmt nach DIN ISO 1628/1 (in Decalin bei 135°C), im Bereich von 1,0 bis 5,0 dl/g aufweist.

3. Heterophasische Propylen-Copolymer-Zusammensetzung nach Anspruch 1 oder 2, wobei das heterophasische Polypropylen-Copolymer aufweist

• eine Gesamtschmelzflussrate (MFRT) (ISO 1133; 230°C; 2,16kg) im Bereich von 0,1 bis 100,0 g/10 min,
• einen Gesamtgehalt an Ethylen-Comonomeren in polymerisierter Form im Bereich von 2,5 bis 15,0 Gew.-%,
• eine erste Glasübergangstemperatur Tg(1) im Bereich von -12°C bis +4°C und/oder eine zweite Glasübergangstemperatur Tg(2) im Bereich von -65°C bis -30°C.

**4.** Heterophasische Propylen-Copolymer-Zusammensetzung nach einem der vorhergehenden Ansprüche, wobei die Matrix (M) des heterophasischen Polypropylen-Copolymers ein statistisches Propylen-Copolymer (R-PP) ist, wobei das statistische Propylen-Copolymer (R-PP) aufweist

• einer Schmelzflussrate MFR2 (230°C), gemessen nach ISO 1133, im Bereich von 0,5 bis 100,0 g/10min und
• einen Ethylen- und/oder C4- bis C8-$\alpha$-Olefin-Comonomergehalt, in polymerisierter Form, im Bereich von 1,0 bis 12,0 Gew.-%.

**5.** Heterophasische Propylencopolymer-Zusammensetzung nach einem der vorhergehenden Ansprüche, wobei das statistische Propylencopolymer (R-PP) und das elastomere Copolymer (E) beide nur aus Ethylen- und Propylenmonomeren hergestellt sind.

**6.** Heterophasische Propylen-Copolymer-Zusammensetzung nach einem der vorhergehenden Ansprüche, wobei das Dien-Comonomer des Elastomers auf Styrolbasis (B) ausgewählt ist aus Butadien und Isopren.

**7.** Heterophasische Propylen-Copolymer-Zusammensetzung nach einem der vorhergehenden Ansprüche, wobei das Elastomer (B) auf Styrolbasis eine

• Schmelzflussrate MFR2 (230 °C, ISO 1133) im Bereich von 2,0 bis 200,0 g/10min und
• eine Shore-A-Härte, gemessen nach ASTM D 2240, im Bereich von 25 bis 65.

**8.** Heterophasische Propylencopolymer-Zusammensetzung nach einem der vorhergehenden Ansprüche, wobei die heterophasische Propylencopolymer-Zusammensetzung aufweist

• einer Schmelzflussrate MFR2 (230 °C, ISO 1133) im Bereich von 0,5 bis 50,0 g/10min und
• einen in Xylol heiß unlöslichen Gehalt im Bereich von 0,10 bis 2,00 Gew.-%.

**9.** Heterophasische Propylencopolymer-Zusammensetzung nach einem der vorhergehenden Ansprüche, wobei die heterophasische Propylencopolymer-Zusammensetzung aufweist

• einem Zugmodul nach ISO 527-1,2 gemessen an einer 50 $\mu$m Gießfolie von unter 350 MPa und
• eine Charpy-Kerbschlagzähigkeit nach ISO 179 (1eA) bei -20°C von mindestens 15,0 kJ/m2, und bei -30°C von mindestens 7,0 kJ/m2.

**10.** Verfahren zur Herstellung der gepfropften heterophasischen Propylencopolymer-Zusammensetzung nach einem der Ansprüche 1 bis 9, wobei das Verfahren ein reaktives Schmelzmodifizierungsverfahren ist, umfassend die Schritte

i) Mischen der Komponente (A), des heterophasischen Polypropylen-Copolymers mit der Komponente (B), dem Elastomer auf Styrolbasis und einer thermisch zersetzenden, freie Radikale bildenden Verbindung (C) und
ii) Extrudieren der Mischung.

**11.** Verfahren nach Anspruch 10, wobei das heterophasische Polypropylen-Copolymer (A) in einem mehrstufigen Verfahren hergestellt wird, das mindestens zwei in Reihe geschaltete Reaktoren umfasst.

**12.** Verfahren nach Anspruch 10 oder 11, wobei die thermisch zersetzende radikalbildende Verbindung (C) ein Peroxid ist.

**13.** Verwendung der gepfropften heterophasischen Polypropylen-Copolymer-Zusammensetzung nach einem der Ansprüche 1 bis 9 zur Herstellung von Gegenständen, ausgewählt aus extrudierten Folien oder Schläuchen, Beuteln, blasgeformten oder spritzgeformten Gegenständen.

**14.** Gegenstände, die mehr als 90 Gew.-% der gepfropften heterophasischen Polypropylen-Copolymer-Zusammensetzung nach einem der Ansprüche 1 bis 9 umfassen.

**15.** Artikel nach Anspruch 14, wobei es sich bei den Artikeln um medizinische Schläuche oder Beutel handelt, die mehr als 90 Gew.-% der gepfropften heterophasischen Polypropylen-Copolymer-Zusammensetzung nach einem der Ansprüche 1 bis 9 umfassen.

**EP 3 615 098 B1**

**Revendications**

1.  Composition de copolymère de propylène hétérophasique greffé, qui comprend

    (A) 30,0 à 95,0 % en poids d'un copolymère de polypropylène hétérophasique ayant un indice de fluage (MFR 230°C/2,16 kg, ISO 1133) de 0,1 à 100 g/10 min et comprenant

    (a1) une matrice (M) qui est un homo- ou co-polymère de polypropylène cristallin et
    (a2) un copolymère élastomère (E) dispersé dans ladite matrice (M)
    et

    (B) 5,0 à 70,0 % en poids d'un élastomère à base de styrène qui est un copolymère séquencé de styrène insaturé contenant au moins un comonomère diène et ayant une teneur en styrène de 5,0 à 50,0 % en poids, mesuré par spectroscopie infrarouge à transformée de Fourier (FTIR),
    et dans laquelle l'élastomère à base de styrène (B) est greffé sur le copolymère de polypropylène hétérophasique de base (A),
    dans laquelle la composition de copolymère de propylène hétérophasique greffé a une résistance au choc, mesurée conformément au test de choc sur barreau entaillé Charpy conformément à la norme ISO 179 (1eA) à -20°C, d'au moins 15,0 kJ/m$^2$, une résistance au choc, mesurée conformément au test de choc sur barreau entaillé Charpy conformément à la norme ISO 179 (1eA) à -30°C, d'au moins 7,0 kJ/m$^2$, et une résistance à l'état fondu F30, mesurée avec le test Rheotens à 200°C conformément à la norme ISO 16790:2005, d'au moins 3,0 cN.

2.  Composition de copolymère de propylène hétérophasique selon la revendication 1, dans laquelle le copolymère de polypropylène hétérophasique a une teneur soluble à froid dans le xylène (XCS), déterminée conformément à la norme ISO 16152 (25°C), dans la plage de 10,0 à 50,0 % en poids, la teneur soluble à froid dans le xylène (XCS) ayant une viscosité intrinsèque (iV), déterminée conformément à la norme DIN ISO 1628/1 (dans la décaline à 135°C), dans la plage de 1,0 à 5,0 dl/g.

3.  Composition de copolymère de propylène hétérophasique selon la revendication 1 ou 2, dans laquelle le copolymère de polypropylène hétérophasique a

    • un indice de fluage total (MFRT) (ISO 1133 ; 230°C ; 2,16 kg) dans la plage de 0,1 à 100,0 g/10 min,
    • une teneur en comonomère éthylène totale, sous forme polymérisée, dans la plage de 2,5 à 15,0 % en poids,
    • une première température de transition vitreuse Tg(1) dans la plage de -12°C à +4°C, et/ou une deuxième température de transition vitreuse Tg(2) dans la plage de -65°C à -30°C.

4.  Composition de copolymère de propylène hétérophasique selon l'une quelconque des revendications précédentes, dans laquelle la matrice (M) du copolymère de polypropylène hétérophasique est un copolymère de propylène aléatoire (R-PP), dans laquelle le copolymère de propylène aléatoire (R-PP) a

    • un indice de fluage MFR$_2$ (230°C), mesuré conformément à la norme ISO 1133, dans la plage de 0,5 à 100,0 g/10 min, et
    • une teneur en comonomères éthylène et/ou $\alpha$-oléfines en C$_4$ à C$_8$, sous forme polymérisée, dans la plage de 1,0 à 12,0 % en poids.

5.  Composition de copolymère de propylène hétérophasique selon l'une quelconque des revendications précédentes, dans laquelle le copolymère de propylène aléatoire (R-PP) et le copolymère élastomère (E) sont tous deux faits de monomères éthylène et propylène uniquement.

6.  Composition de copolymère de propylène hétérophasique selon l'une quelconque des revendications précédentes, dans laquelle le comonomère diène de l'élastomère à base de styrène (B) est choisi parmi le butadiène et l'isoprène.

7.  Composition de copolymère de propylène hétérophasique selon l'une quelconque des revendications précédentes, dans laquelle l'élastomère à base de styrène (B) a

    • un indice de fluage MFR$_2$ (230°C, ISO 1133) dans la plage de 2,0 à 200,0 g/10 min, et
    • une dureté Shore A, mesurée conformément à la norme ASTM D 2240, dans la plage de 25 à 65.

8. Composition de copolymère de propylène hétérophasique selon l'une quelconque des revendications précédentes, dans laquelle la composition de copolymère de propylène hétérophasique a

   • un indice de fluage MFR$_2$ (230°C, ISO 1133) dans la plage de 0,5 à 50,0 g/10 min, et
   • une teneur insoluble à chaud dans le xylène dans la plage de 0,10 à 2,00 % en poids.

9. Composition de copolymère de propylène hétérophasique selon l'une quelconque des revendications précédentes, dans laquelle la composition de copolymère de propylène hétérophasique a

   • un module de traction, conformément à la norme ISO 527-1,2, mesuré sur un film coulé de 50 $\mu$m, inférieur à 350 MPa, et
   • une résistance au choc sur barreau entaillé Charpy, conformément à la norme ISO 179 (1eA), à -20°C d'au moins 15,0 kJ/m$^2$, et à -30°C d'au moins 7,0 kJ/m$^2$.

10. Procédé pour produire la composition de copolymère de propylène hétérophasique greffé selon l'une quelconque des revendications 1 à 9, lequel procédé est un procédé de modification à l'état fondu réactive comprenant les étapes de

    i) mélange du composant (A), le copolymère de polypropylène hétérophasique, avec le composant (B), l'élastomère à base de styrène, et un composé formant des radicaux libres se décomposant thermiquement (C), et
    ii) extrusion dudit mélange.

11. Procédé selon la revendication 10, dans lequel le copolymère de polypropylène hétérophasique (A) est produit dans un procédé à étages multiples comprenant au moins deux réacteurs connectés en série.

12. Procédé selon la revendication 10 ou 11, dans lequel le composé formant des radicaux libres se décomposant thermiquement (C) est un peroxyde.

13. Utilisation de la composition de copolymère de propylène hétérophasique greffé selon l'une quelconque des revendications 1 à 9, pour produire des articles choisis parmi des films ou tubes extrudés, des poches, des articles moulés par soufflage ou moulés par injection.

14. Articles comprenant plus de 90 % en poids de la composition de copolymère de propylène hétérophasique greffé selon l'une quelconque des revendications 1 à 9.

15. Articles selon la revendication 14, dans lesquels les articles sont des tubes ou poches à usage médical comprenant plus de 90 % en poids de la composition de copolymère de propylène hétérophasique greffé selon l'une quelconque des revendications 1 à 9.

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 2048185 A
- EP 1564231 A1
- WO 9924478 A
- WO 9924479 A
- WO 0068315 A
- EP 887379 A
- EP 887380 A
- EP 887381 A
- EP 991684 A
- EP 0887379 A
- WO 9212182 A
- WO 2004000899 A
- WO 2004111095 A
- WO 2003000757 A
- WO 2003000754 A
- WO 2004029112 A
- WO 2007137853 A
- WO 2012007430 A
- EP 2610271 A
- EP 261027 A
- EP 2610272 A
- WO 006831 A

**Non-patent literature cited in the description**

- **HANS ZWEIFEL.** Plastic Additives Handbook. 2001
- **SINGH, G. ; KOTHARI, A. ; GUPTA, V.** *Polymer Testing,* 2009, vol. 28 (5), 475
- **ZHOU, Z. ; KUEMMERLE, R. ; QIU, X. ; REDWINE, D. ; CONG, R. ; TAHA, A. ; BAUGH, D. ; WINNIFORD, B.** *J. Mag. Reson.,* 2007, vol. 187, 225
- **BUSICO, V. ; CARBONNIERE, P. ; CIPULLO, R. ; PELLECCHIA, R. ; SEVERN, J. ; TALARICO, G.** *Macromol. Rapid Commun.,* 2007, vol. 28, 1128
- **CHENG, H. N.** *Macromolecules,* 1984, vol. 17, 1950
- **L. RESCONI ; L. CAVALLO ; A. FAIT ; F. PIEMONTESI.** *Chem. Rev.,* 2000, vol. 100 (4), 1253
- **W-J. WANG ; S. ZHU.** *Macromolecules,* 2000, vol. 33, 1157
- **WANG, W-J. ; ZHU, S.** *Macromolecules,* 2000, vol. 33, 1157
- **KAKUGO, M. ; NAITO, Y. ; MIZUNUMA, K. ; MIYATAKE, T.** *Macromolecules,* 1982, vol. 15, 1150
- **M. H. WAGNER.** Rheotens-Mastercurves and Drawability of Polymer Melts. *Polymer Engineering and Sience,* vol. 36, 925-935